# EUROPEAN PATENT APPLICATION

(11) **EP 3 146 989 A1**
(43) Date of publication of application: **29.03.2017**
(21) Application number: 15799137.3
(22) Date of filing: 28.05.2015
(51) Int. Cl.: A61M 11/02, A61M 15/00, B65D 83/00, F24F 6/00, F24F 6/14

(54) **HUMIDIFYING DEVICE**

(30) Priority: 28.05.2014 JP 2014109889
(71) Applicant: Metran Co., Ltd., Kawaguchi-shi, Saitama 332-0015 (JP)
(72) Inventor: OSADA Yasuo, Kawaguchi-shi Saitama 332-0015 (JP); NITTA Kazufuku, Kawaguchi-shi Saitama 332-0015 (JP); NITTA Dan, Kawaguchi-shi Saitama 332-0015 (JP); NAKANE Shinichi, Kawaguchi-shi Saitama 332-0015 (JP)
(74) Representative: TBK
(86) International application number: PCT/JP2015/065430
(87) International publication number: WO 2015/182713

(57) **Abstract**

A nebulizer includes: a suction mechanism configured to suck a liquid for humidification from a water bottle containing the liquid and form an aerosol of the sucked liquid by a negative pressure produced by an oxygen gas jetted from a gas jetting part of a nozzle member; a liquid receptor configured to store the liquid residing in the form of droplets into which the aerosol turns; and a receptor-side aerosol forming member configured to suck the liquid from the liquid receptor and form an aerosol of the sucked liquid by the negative pressure. Consequently, germs are prevented from getting into the liquid in a container during use, and the liquid is prevented from dripping when the container is replaced.

## Description

### Technical Field

The present invention relates to a humidifying device, and more particularly to a humidifying device for humidifying a gas such as air and oxygen supplied to a patient.

### Background Art

Supplying oxygen to a patient has conventionally been practiced in hospitals and the like. Oxygen generated by an oxygen cylinder and the like is supplied to a patient by using a mask and the like. The oxygen supplied from an oxygen cylinder and the like contains little moisture. In supplying the oxygen to an airway such as the nasal cavity of the patient, drying of the airway therefore needs to be prevented. A humidifying device is arranged on the way of the oxygen supply tube, so that humidified oxygen is supplied.

Among commonly known humidifying devices used to humidify oxygen is a nebulizer. This humidifying device is configured to include: a water bottle (container) which contains a liquid such as a medicine-dissolved solution, sterile water, purified water, distilled water, a physiological saline solution, and the like; a dedicated humidifying device adaptor (nebulizer adaptor) which is connected to the water bottle; and the like. The nebulizer adaptor is an adaptor configured to jet out an oxygen gas from an orifice formed in a nozzle member, thereby sucking up the sterile water or the like contained in the water bottle from a suction hole arranged near the orifice and sucking in air, and forming a fine aerosol of the sucked sterile water or the like to humidify a gas containing a high concentration of oxygen so that the humidified gas can be supplied to a patient.

If the water bottle of the sterile water or the like becomes empty, the water bottle needs to be replaced. Many nebulizer adaptors are therefore configured so that the water bottle can be replaced. A conventional nebulizer is configured to include a water supply pipe for sucking up the sterile water or the like from the water bottle to the nebulizer adaptor, and a drain tube for returning water accumulated in the nebulizer adaptor to the water bottle (for example, see Patent Literature 1).

### Citation List

### Patent Literature

[Patent Literature 1] Japanese Patent Application Laid-Open No. 2012-071011

### Summary of Invention

### Technical Problem

Conventionally, the sterile water or the like sucked up from the water bottle to the nebulizer adaptor is formed into an aerosol to humidify the oxygen gas, and supplied to the patient with the oxygen gas. However, the sucked sterile water or the like does not all become the aerosol, but resides in part inside the nebulizer adaptor in the form of water droplets. The sterile water or the like residing in the nebulizer adaptor therefore has needed to be returned by using the drain tube or the like.

Depending on conditions such as the state of the patient and the supply amount of oxygen, the sterile water or the like in the water bottle is consumed quickly, and the water bottle has needed to be replaced with a new one frequently. The nebulizer adaptor is thus configured so that the water bottle can be replaced. In replacing the water bottle in the presence of the drain tube described above, the drain tube has needed to be reconnected from the old water bottle to the new water bottle. Since water droplets from the nebulizer adaptor flow constantly through the drain tube as described above, there has been a problem that the water drips from the drain tube when the drain tube is reconnected from the old water bottle to the new water bottle.

The sterile water or the like sucked up from the water bottle to the nebulizer adaptor is formed into an aerosol and mixed with the air taken in from inside the room. Here, germs in the room can also get in. Non-aerosol components of the sterile water or the like mixed with the germs included in the room air reside inside the nebulizer adaptor in the form of droplets. Since such sterile water or the like accumulated in the nebulizer adaptor is let into the water bottle as a drain, the germs in the sterile water or the like enter the water bottle. The water bottle originally contains a liquid, such as sterile water, with little germs in it. Such water bottles are commercially available. However, there has been a problem that if the sterile water or the like returns to the water bottle as a drain, germs can get into the liquid, such as sterile water, in the water bottle.

A heater device may be interposed between the water bottle and the nebulizer adaptor to heat the sterile water or the like while the sterile water or the like contained in the water bottle is sucked up. In such a case, the heater device is configured so that the sterile water or the like makes direct contact with and passes through the interior of the heater device. Therefore, there has been a problem that the heater device or its components need to be subjected to sterilization treatment each time the patient changes.

The present invention has been achieved in view of the foregoing problems, and an object thereof is to provide a humidifying device which prevents germs from getting into the liquid, such as sterile water, in the container, such as a water bottle, during use, and prevent the liquid from dripping when the container is replaced.

### Solution to Problem

(1) To solve the foregoing problems, a humidifying device according to the present invention is a humidifying device including: a suction mechanism configured to suck a liquid for humidification from a container containing the liquid by a negative pressure produced by a gas jetted from a gas jetting part of a nozzle member; a liquid receptor configured to store the liquid sucked by the suction mechanism; and a receptor-side aerosol forming member configured to suck the liquid from the liquid receptor and form an aerosol of the sucked liquid by the negative pressure by using a receptor-side suction passage.
(2) The humidifying device according to the present invention is the humidifying device according to (1) above, wherein the suction mechanism includes a container-side suction member configured to suck the liquid from the container by the negative pressure produced by the gas jetted from the gas jetting part of the nozzle member.
(3) The humidifying device according to the present invention is the humidifying device according to (2) above, wherein: the container-side suction member of the suction mechanism is a container-side aerosol forming member configured to suck the liquid from the container and form an aerosol of the sucked liquid by the negative pressure produced by the gas jetted from the gas jet part of the nozzle member; and the liquid receptor stores at least the liquid discharged from the container-side aerosol forming member.
(4) The humidifying device according to the present invention is the humidifying device according to (1) above, including a suction force control mechanism configured to make capability of the suction mechanism to suck the liquid of the container variable by using a rise or fall of a liquid surface of the liquid stored in the liquid receptor.
(5) The humidifying device according to the present invention is the humidifying device according to (4) above, wherein: the suction mechanism includes a discharge port configured to discharge the liquid sucked from the container; and the suction force control mechanism includes a cutoff member configured to move between a cutoff position in which to cut off the discharge port from a flow of the gas jetted from the gas jetting part and a retracted position in which to be retracted from the cutoff position, a floating member configured to float up and down according to the rise or fall of the liquid surface of the liquid stored in the liquid receptor, and a connection member configured to connect the floating member and the cutoff member and cause the cutoff member to move with floating of the floating member.
(6) The humidifying device according to the present invention is the humidifying device according to (5) above, wherein the cutoff member moves between the cutoff position and the retracted position by oscillating about an oscillation shaft.
(7) The humidifying device according to the present invention is the humidifying device according to (4) above, wherein: the suction mechanism includes a container-side suction passage configured to suck and guide the liquid from the container; and the suction force control mechanism includes an air opening port that is capable of opening a middle part of the container-side suction passage to an air side, and an air control valve configured to open and close the air opening port according to the rise or fall of the liquid surface of the liquid stored in the liquid receptor.
(8) The humidifying device according to the present invention is the humidifying device according to (4) above, wherein: the suction mechanism includes a container-side suction passage configured to suck and guide the liquid from the container; and the suction force control mechanism includes a container-side suction passage control valve configured to control passing of the liquid of the container-side suction passage according to the rise or fall of the liquid surface of the liquid stored in the liquid receptor.
(9) The humidifying device according to the present invention is the humidifying device according to (1) above, wherein the suction mechanism includes a communication passage configured to communicate the liquid of the container to the receptor-side suction passage of the receptor-side aerosol forming member, so that the suction mechanism sucks the liquid from the container by using a suction force of the receptor-side aerosol forming member.
(10) The humidifying device according to the present invention is the humidifying device according to (9) above, including a suction force control mechanism configured to make capability of the suction mechanism to suck the liquid of the container variable by using a rise or fall of a liquid surface of the liquid stored in the liquid receptor.
(11) The humidifying device according to the present invention is the humidifying device according to (10) above, wherein the suction force control mechanism includes a control member that is arranged on the receptor-side suction passage and configured to control a suction amount of the liquid of the liquid receptor by using the rise or fall of the liquid surface of the liquid stored in the liquid receptor, and the suction force control mechanism accelerates suction of the liquid of the container through the communication passage by suppressing the suction amount of the liquid of the liquid receptor by the control member.
(12) The humidifying device according to the present invention is the humidifying device according to (11) above, wherein the control member of the suction force control mechanism includes: an opening and closing member configured to move between a closing position where a liquid suction port of the liquid of the liquid receptor in the receptor-side aerosol forming member is closed and an opening position where the liquid suction port is opened; a floating member configured to float up and down according to the rise or fall of the liquid surface of the liquid stored in the liquid receptor; and a connection member configured to connect the floating member and the opening and closing member and cause the opening and closing member to move with floating of the floating member.
(13) The humidifying device according to the present invention is the humidifying device according to (1) above, including a second liquid receptor configured to receive the liquid above a bottom surface of the liquid receptor, wherein the receptor-side aerosol forming member sucks the liquid guided by the second liquid receptor and form an aerosol of the sucked liquid.
(14) The humidifying device according to the present invention is the humidifying device according to (13) above, wherein the second liquid receptor is submerged in the liquid stored in the liquid receptor.
(15) The humidifying device according to the present invention is the humidifying device according to (13) above, wherein the second liquid receptor also serves as a restriction member configured to restrict impingement of the gas jetted from the gas jetting part of the nozzle member on a liquid surface of the liquid receptor.
(16) The humidifying device according to the present invention is the humidifying device according to (1) above, including a heating mechanism configured to heat at least part of the liquid of the liquid receptor or the liquid in the receptor-side aerosol forming member.
(17) The humidifying device according to the present invention is the humidifying device according to (1) above, including an adaptor that is detachably attached to the container, wherein the adaptor includes at least the receptor-side aerosol forming member, the liquid receptor, and the suction mechanism.
(18) The humidifying device according to the present invention is the humidifying device according to (1) above, the humidifying device humidifying and sending the gas containing oxygen to a patient, the humidifying device including a sending unit configured to send out a mixture of the gas and the aerosol.
(19) The humidifying device according to the present invention is the humidifying device according to (1) above, including the container.

The humidifying device according to the present invention includes: the container-side aerosol forming member configured to suck the liquid for humidification from the container containing the liquid and form an aerosol of the sucked liquid by a negative pressure produced by a gas jetted from an orifice of the nozzle member; the liquid receptor configured to store the liquid residing in the form of droplets into which the aerosol turns; and the receptor-side aerosol forming member configured to suck the liquid from the liquid receptor and form an aerosol of the sucked liquid again by the negative pressure. A drain tube for returning the liquid, such as sterile water, accumulated in the humidifying device to the container, such as a water bottle, therefore does not need to be provided. This facilitates replacement of the container, and solves the conventional problem that the liquid drips from the drain tube when the container is replaced.

The humidifying device according to the present invention does not need to include a drain tube for returning a liquid, such as sterile water, accumulated in a nebulizer adaptor to the container, such as a water bottle. Since the liquid, such as sterile water, to be a drain containing germs in the room will not return to the container, such as a water bottle, the problem that germs can get into the liquid in the container is solved.

The humidifying device according to the present invention includes: the cutoff member configured to oscillate about the oscillation shaft between the cutoff position in which to cut off an ejection port of the container-side aerosol forming member from the orifice and the retracted position in which to be retracted from the cutoff position; the floating member configured to float up and down according to the rise or fall of the liquid surface of the liquid stored in the liquid receptor; and the connection member configured to connect the floating member to the cutoff member with the connection shaft and cause the cutoff member to oscillate with the floating of the floating member. If the amount of the liquid stored in the liquid receptor increases, the humidifying device can thus stop sucking the liquid from the container. This can prevent the amount of the liquid stored in the liquid receptor from continuing to increase.

The humidifying device according to the present invention includes: the container-side suction member configured to suck the liquid for humidification from the container containing the liquid by the negative pressure produced by the gas jetted from the orifice of the nozzle member; the liquid receptor configured to store the liquid sucked by the container-side suction member; and an aerosol forming member configured to suck the liquid from the liquid receptor and form an aerosol of the sucked liquid by the negative pressure. A drain tube for returning the liquid, such as sterile water, accumulated in the humidifying device to the container, such as a water bottle, therefore does not need to be provided. This facilitates the replacement of the container, and solves the conventional problem that the liquid drips from the drain tube when the container is replaced.

The humidifying device according to the present invention does not need to include a drain tube for returning the liquid, such as sterile water, accumulated in the nebulizer adaptor to the container, such as a water bottle. Since the liquid, such as sterile water, to be a drain containing germs in the room will not return to the container, such as a water bottle, the drawback that germs can get into the liquid in the container is solved.

In the humidifying device according to the present invention, if the container-side suction member continues sucking the liquid in the container, such as a water bottle, and the liquid stored in the liquid receptor increases, an outlet of the container-side suction member is submerged in the liquid. This stops the function of sucking up the liquid by the container-side suction member. The liquid stored in the liquid receptor can thus be prevented from overflowing into a horizontal projection portion. As the aerosol forming member sucks up the liquid stored in the liquid receptor, the outlet of the container-side suction member emerges from the liquid surface of the liquid. The function of sucking up the liquid by the container-side suction member is thereby restored.

The humidifying device according to the present invention includes: the cutoff member configured to oscillate about the oscillation shaft between the cutoff position in which to cut off the outlet of the container-side suction member from the orifice and the retracted position in which to be retracted from the cutoff position; the floating member configured to float up and down according to the rise or fall of the liquid surface of the liquid stored in the liquid receptor; and the connection member configured to connect the floating member to the cutoff member with the oscillating shaft and cause the cutoff member to oscillate with the floating of the floating member. If the amount of the liquid stored in the liquid receptor increases, the humidifying device can thus stop sucking the liquid from the container. This can prevent the amount of the liquid stored in the liquid receptor from continuing to increase.

### Advantageous Effects of Invention

According to the humidifying device of the present invention, excellent effects that germs can be prevented from getting into the liquid, such as sterile water, in the container, such as a water bottle, during use, and the liquid can be prevented from dripping when the container is replaced can be provided.

### Brief Description of Drawings

FIG. 1 is a cross-sectional view for describing a configuration of a nebulizer according to a first embodiment of the present invention.
FIG. 2 is a side view for describing an air suction port formed by a window of an adjustment dial and a window of an erected projection portion.
FIGs. 3(A), 3(B), and 3(C) are cross-sectional views and bottom views showing examples of a relationship between an ejection port of a liquid of a container-side aerosol forming member and an ejection port of a liquid of a receptor-side aerosol forming member.
FIG. 4 is a cross-sectional view showing a distance from an orifice of a nozzle member in a jetting direction of an oxygen gas and a graph showing as a general concept a relationship between the distance and a negative pressure at the part of an ejection port of a liquid.
FIG. 5 is a side view and a bottom view showing a distance from a center of the orifice of the nozzle member, and a graph showing as a general concept a relationship between the distance and the negative pressure at the part of the ejection port of a liquid.
FIG. 6 is a side view showing an example of an oxygen flowmeter.
FIG. 7 is a cross-sectional view for describing a configuration of a nebulizer according to a second embodiment of the present invention.
FIG. 8 is a conceptual diagram for describing a structure of a valve included in a nebulizer adaptor that constitutes a nebulizer according to a third embodiment of the present invention.
FIG. 9 is an enlarged view for describing the structure of the valve.
FIG. 10 is a perspective view showing a positional relationship between an outlet of a container-side suction member, an ejection port of an aerosol forming member, and the orifice of the nozzle member.
FIG. 11 is a perspective view of the valve.
FIG. 12 is a cross-sectional view showing the positional relationship between the outlet of the container-side suction member, the ejection port of the aerosol forming member, and the orifice of the nozzle member.
FIG. 13(A) is a conceptual diagram for describing a structure of a valve included in a nebulizer adaptor that constitutes a nebulizer according to a fourth embodiment of the present invention, and FIG. 13(B) is an enlarged view showing essential parts of the structure shown in FIG. 13(A).
FIG. 14(A) is a conceptual diagram for describing a structure of a valve included in a nebulizer adaptor that constitutes a nebulizer according to a fifth embodiment of the present invention, and FIG. 14(B) is a top view showing essential parts of the structure shown in FIG. 14(A).
FIG. 15 is a conceptual diagram for describing a structure of a valve included in a nebulizer adaptor that constitutes a nebulizer according to a sixth embodiment of the present invention.
FIG. 16 is a perspective view of the valve.
FIG. 17(A) is a cross-sectional view of a nebulizer adaptor constituting a nebulizer according to a seventh embodiment of the present invention at full liquid time, and FIG. 17(B) is a top view showing essential parts of the structure shown in FIG. 17(A).
FIG. 18(A) is a cross-sectional view of the nebulizer adaptor according to the seventh embodiment when the liquid surface is low, and FIG. 18(B) is a cross-sectional view of a structure of essential parts, showing an application example of the nebulizer adaptor according to the seventh embodiment.
FIG. 19 is a cross-sectional view of the application example of the nebulizer adaptor according to the seventh embodiment at full liquid time.
FIG. 20 is a cross-sectional view of the application example when the liquid surface is low.
FIG. 21(A) is a cross-sectional view of a structure of essential parts of a nebulizer adaptor constituting a nebulizer according to an eighth embodiment of the present invention when the liquid surface is low, and FIG. 21(B) is a cross-sectional view of the structure of the essential parts at full liquid time.
FIG. 22(A) is a cross-sectional view of a structure of essential parts of a nebulizer adaptor constituting a nebulizer according to a ninth embodiment of the present invention when the liquid surface is low, and FIG. 22(B) is a cross-sectional view of the structure of the essential parts at full liquid time.
FIG. 23(A) is an enlarged cross-sectional view of essential parts of a nebulizer adaptor constituting a nebulizer according to a tenth embodiment of the present invention at full liquid time, FIG. 23(B) is a sectional side view taken along the line B-B of FIG. 23(A), and FIG. 23(C) is an enlarged cross-sectional view of the essential parts when the liquid surface is low.
FIG. 24(A) is a cross-sectional plan view showing a suction force control mechanism of the nebulizer adaptor, FIG. 24(B) is a cross-sectional front view of the suction force control mechanism, FIG. 24(C) is a left side view showing only an air control valve of the suction force control mechanism, and FIG. 24(D) is a right side view showing only the air control valve.
FIG. 25 is a cross-sectional front view showing an open state of the suction force control mechanism.
FIG. 26 is a cross-sectional view of a nebulizer adaptor constituting a nebulizer according to an eleventh embodiment of the present invention at full liquid time.
FIG. 27 is a cross-sectional view of the nebulizer adaptor according to the eleventh embodiment when the liquid surface is low.
FIG. 28 is a cross-sectional view of the nebulizer adaptor according to the eleventh embodiment at balanced time.
FIG. 29(A) is a cross-sectional plan view showing a suction force control mechanism of the nebulizer adaptor, FIG. 29(B) is a cross-sectional front view of the suction force control mechanism, FIG. 29(C) is a left side view showing only control members of the suction force control mechanism, and FIG. 29(D) is a right side view showing only the control members.
FIG. 30 is a cross-sectional view showing an application example of the nebulizer adaptor according to the eleventh embodiment.
FIG. 31 is a cross-sectional view showing an application example of the nebulizer adaptor according to the eleventh embodiment at liquid full time.
FIG. 32 is a cross-sectional view showing the application example of the nebulizer adaptor according to the eleventh embodiment when the liquid surface is low.

### Description of Embodiments

A nebulizer according to each of embodiments of the present invention will be described below with reference to the drawings.

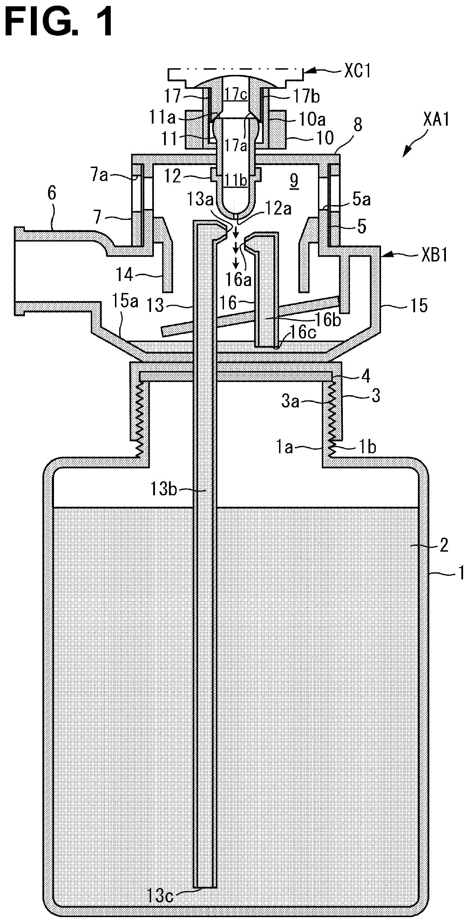

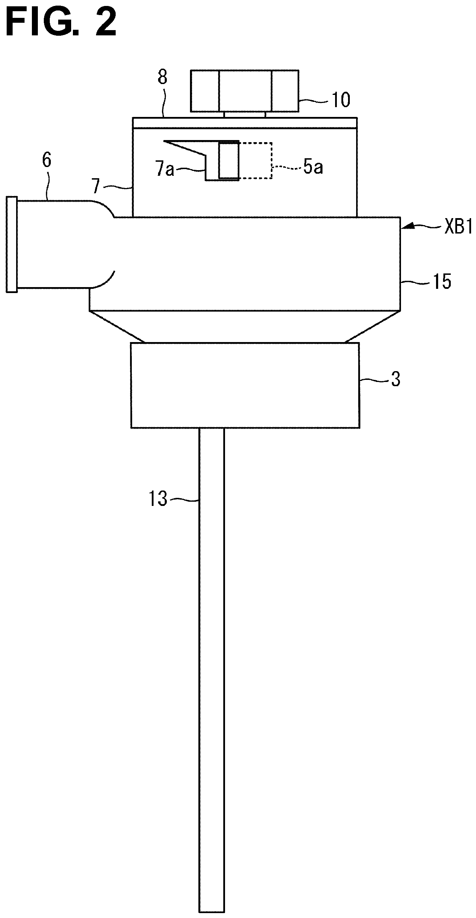

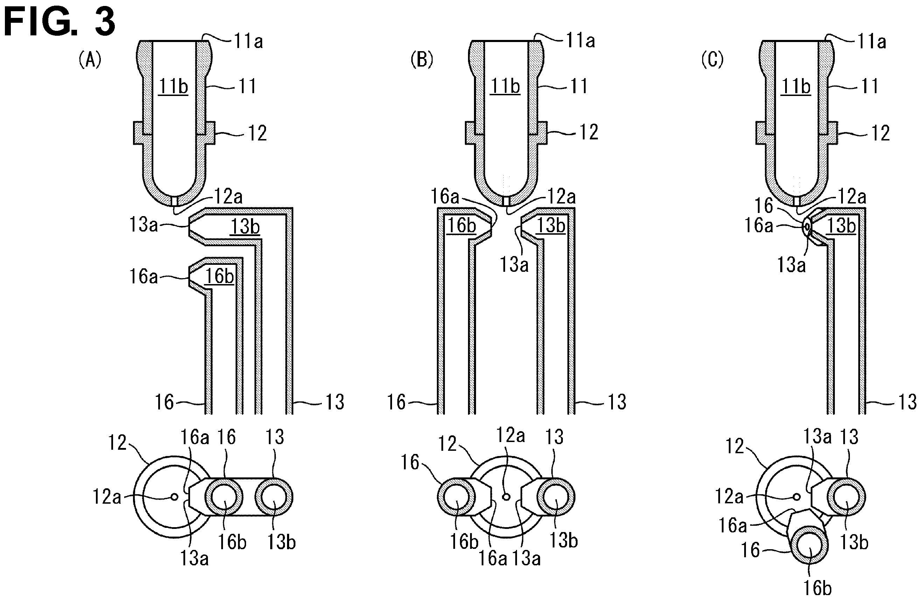

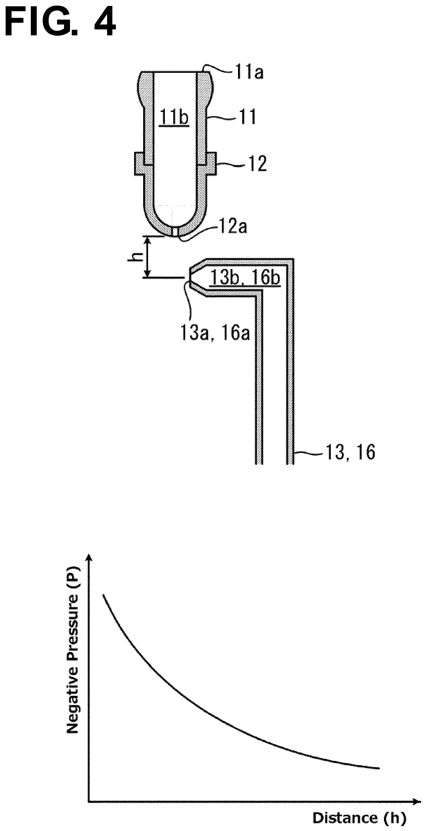

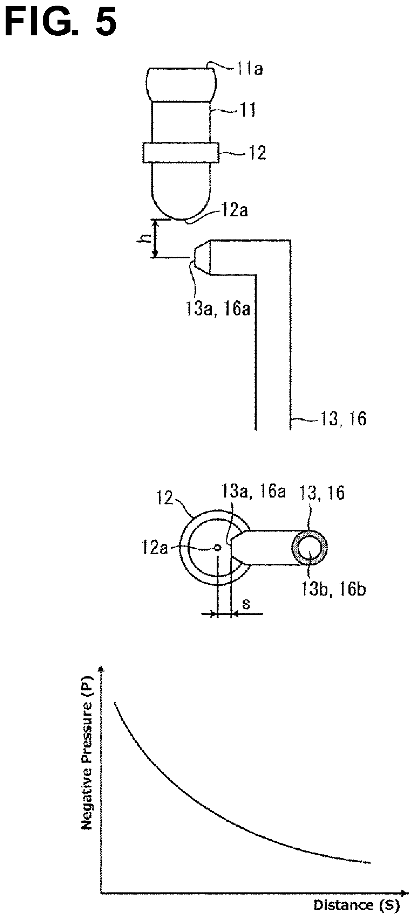

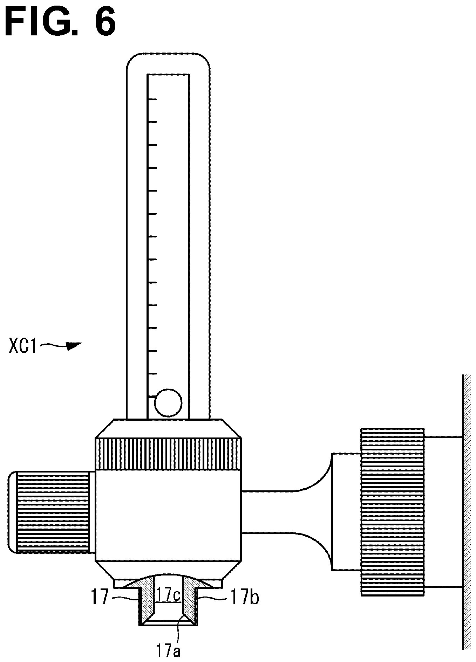

[First Embodiment] Initially, a configuration of a nebulizer XA1 according to a first embodiment will be described with reference to FIGs. 1 to 6. FIG. 1 is a cross-sectional view for describing a configuration of the nebulizer XA1 according to the first embodiment of the present invention. FIG. 2 is a side view for describing an air suction port formed by a window 7a of an adjustment dial 7 and a window 5a of an erected projection portion 5. FIG. 3 is a diagram showing examples of a positional relationship between an ejection port 13a of a liquid 2 of a container-side aerosol forming member 13 and an ejection portion 16a of the liquid 2 of a receptor-side aerosol forming member 16. FIG. 4 is a cross-sectional view showing a distance h from an orifice (gas jetting part) 12a of a nozzle member 12 in a jetting direction of oxygen gas (oxygen-containing gas) and a graph showing as a general concept a relationship between the distance h and a negative pressure at the part of the ejection ports 13a and 16a of the liquid 2. FIG. 5 is a side view and a top view showing a distance s from a center of the orifice 12a of the nozzle member 12, and a graph showing as a general concept a relationship between the distance s and the negative pressure at the part of the ejection ports 13a and 16a of the liquid 2. FIG. 6 is a side view showing an example of an oxygen flowmeter XC1. In these diagrams and subsequent diagrams, some components are omitted as appropriate for simplification.

The nebulizer (humidifying device) XA1 shown in FIG. 1 includes a nebulizer adaptor XB1 and a water bottle (container) 1. The water bottle 1 contains the liquid 2 such as sterile water. An opening portion 1a is arranged on the top of the water bottle 1. A screw portion 1b intended for connection with the nebulizer adaptor XB1 is arranged on an outer peripheral side surface of the opening portion 1a.

A cap 3 to be connected with the foregoing water bottle 1 is arranged on a lower part of the nebulizer adaptor XB1. A screw portion 3a to be threadedly engaged with the screw portion 1b arranged on the outer peripheral side surface of the opening portion 1a of the foregoing water bottle 1 is arranged on an inner side surface of the cap 3.

A gasket 4 is arranged inside the cap 3. If the screw portion 1b and the screw portion 3a are threadedly engaged to connect the nebulizer adaptor XB1 and the water bottle 1, the gasket 4 functions to avoid liquid leak from the connection.

An erected projection portion 5 of cylindrical shape is formed on the nebulizer adaptor XB1 in a direction (perpendicular direction) that becomes vertical when the nebulizer XA1 is positioned upright. This erected projection portion 5 is provided with an oxygen gas supply system. A horizontal projection portion 6 of cylindrical shape is formed on a side of the nebulizer adaptor XB1 away from the erected projection portion 5, in a direction (horizontal direction) that becomes lateral when the nebulizer XA1 is positioned upright. The horizontal projection portion 6 is configured to be able to send out a mixture of air, an oxygen gas, and an aerosol to a patient.

A rotatable adjustment dial 7 is arranged outside the erected projection portion 5. The top of the erected projection portion 5 is closed by a top plate 8. A closed space 9 constituted by the projection portion 5 and the top plate 8 is thus formed inside the erected projection portion 5.

A terminal 11 to which a nut 10 is attached is fitted into the top plate 8. The nut 10 is connected to an outlet member 17 of the oxygen flowmeter XC1 such as shown in FIG. 6, whereby the oxygen gas is supplied.

More specifically, a screw portion 10a arranged on the nut 10 and a screw member 17b arranged on the outlet member 17 of the oxygen flowmeter XC1 are threadedly engaged, whereby a connection portion 17a arranged on the outlet member 17 of the oxygen flowmeter XC1 and a connection portion 11a arranged on the terminal 11 are put in close contact with each other. A flow hole 17c arranged in the outlet member 17 of the oxygen flowmeter XC1 and a flow hole 11b arranged in the terminal 11 communicate with each other to supply the oxygen gas from the oxygen flowmeter XC1 to the terminal 11.

As shown in FIG. 2, windows 7a are formed in a side surface of the adjustment dial 7. Windows 5a are formed in a side surface of the erected projection portion 5 in positions opposed to the windows 7a. The windows 7a and the windows 5a form openings which have a function as an air suction hole.

If the adjustment dial 7 is rotated to oppose the windows 7a to the windows 5a, openings communicating with the closed space 9 are formed. More specifically, the rotation position of the adjustment dial 7 can be adjusted to adjust the area of the openings to the closed space 9, whereby the amount of intake air can be adjusted.

Returning to FIG. 1, a description will be given. A nozzle-like diffuser 14 is arranged inside the erected projection portion 5 of cylindrical shape. This diffuser 14 is not formed to spread out in a fan shape. A top end portion of the diffuser 14 is formed in a tapered shape, and portions below the tapered portion are formed in a straight pipe shape. The nozzle member 12 is arranged in the tapered portion of the diffuser 14.

With the nozzle member 12 and the diffuser 14 arranged as described above, the oxygen gas jetted from the orifice 12a of the nozzle member 12 passes through the diffuser 14 at high speed. The air residing in the closed space 9 is thus sucked to flow toward the diffuser 14. Here, air is sucked according to the area of the openings formed by the windows 7a and the windows 5a formed corresponding to the rotation position of the adjustment dial 7, and passes through the diffuser 14.

The nozzle member 12 is fitted onto the end of the terminal 11 on the water bottle 1 side. The orifice 12a is formed in the extremity of the nozzle member 12. The ejection port 13a of the container-side aerosol forming member 13 is arranged near the orifice 12a of the nozzle member 12. Since the nozzle member 12 and the container-side aerosol forming member 13 have different functions, the members may be configured as two different members and may be combined with each other.

However, the positional relationship between the oxygen gas jetted from the orifice 12a of the nozzle member 12 and the ejection port 13a for ejecting the liquid 2 is subtle and difficult to adjust. Therefore, the nozzle member 12 and the container-side aerosol forming member 13 are preferably integrally configured.

Similarly, the nozzle member 12 and the receptor-side aerosol forming member 16 may be configured as two different members and may be combined with each other. However, the positional relationship between the oxygen gas jetted from the orifice 12a of the nozzle member 12 and the ejection port 16a for ejecting the liquid 2 is subtle and difficult to adjust. Therefore, the nozzle member 12 and the receptor-side aerosol forming member 16 are preferably integrally configured. In other words, the nozzle member 12, the container-side aerosol forming member 13, and the receptor-side aerosol forming member 16 are preferably integrally configured.

The container-side aerosol forming member 13 is arranged inside the diffuser 14 which is arranged in a housing 15 of the nebulizer adaptor XB1. The ejection port 13a of the liquid 2 is formed near the orifice 12a of the nozzle member 12. A container-side suction passage 13b for sucking up the liquid 2 is formed to be continued from the ejection port 13a. The lower end of the container-side suction passage 13b extends to near the inner bottom of the water bottle 1. A liquid suction port 13c at the lower end is inserted in the liquid 2 such as sterile water, and can efficiently suck up the liquid 2 such as sterile water. More specifically, the container-side aerosol forming member 13 serves as a suction mechanism for sucking the liquid 2 from the water bottle 1 containing the liquid for humidification by a negative pressure produced by the gas.

A liquid receptor 15a is arranged in a lower part of the housing 15 of the nebulizer adaptor XB1. The liquid 2 from the ejection port 13a of the container-side aerosol forming member 13 is formed into an aerosol by the negative pressure produced by the oxygen gas jetted from the orifice 12a of the nozzle member 12, and humidifies the oxygen gas. The mixture of the air, the oxygen gas, and the aerosol can be sent out to the patient from the horizontal projection portion 6. The liquid receptor 15a can store the liquid 2 that remains inside the nebulizer adaptor XB1 in the form of droplets without being guided to the outside, other than the moisture of the liquid 2 guided to the outside.

The receptor-side aerosol forming member 16 is further formed inside the diffuser 14 which is arranged in the housing 15 of the nebulizer adaptor XB1. The ejection port 16a of the liquid 2 of the receptor-side aerosol forming member 16 is formed near the orifice 12a of the nozzle member 12. A receptor-side suction passage 16b for sucking up the liquid 2 is formed to be continued from the ejection port 16a. The lower end of the receptor-side suction passage 16b extends to near the bottom of the foregoing liquid receptor 15a, where a liquid suction port 16c is arranged.

The liquid 2 that is not guided to the outside and accumulated as droplets in the liquid receptor 15a in the lower part of the housing 15 of the nebulizer adaptor XB1, other than the moisture of the liquid 2 guided to the outside, is sucked from the liquid suction port 16c. The liquid 2 passes through the receptor-side suction passage 16b and is ejected from the ejection port 16a of the liquid 2 of the receptor-side aerosol forming member 16 in the form of aerosol. The aerosol is mixed with the liquid 2 ejected from the foregoing container-side aerosol forming member 13 in an aerosol form, and sent out from the horizontal projection portion 6 to the patient as a mixture of the air, the oxygen gas, and the aerosol.

The positional relationship between the ejection port 13a of the liquid 2 of the container-side aerosol forming member 13 and the ejection port 16a of the liquid 2 of the receptor-side aerosol forming member 16 is such that the receptor-side aerosol forming member 16 is placed in a position to not interfere with the formation of the aerosol and the humidification of the oxygen gas by the liquid 2 from the ejection port 13a of the liquid 2 of the container-side aerosol forming member 13.

For example, FIG. 3(A) shows the ejection port 16a of the liquid 2 of the receptor-side aerosol forming member 16, arranged under the ejection port 13a of the liquid 2 of the container-side aerosol forming member 13. FIG. 3(B) shows the ejection port 16a of the liquid 2 of the receptor-side aerosol forming member 16, arranged in a position opposite to the ejection port 13a of the liquid 2 of the container-side aerosol forming member 13. FIG. 3(C) shows the ejection port 13a of the liquid 2 of the container-side aerosol forming member 13 and the ejection port 16a of the liquid 2 of the receptor-side aerosol forming member 16 which are in the same height position. When seen from below, the ejection ports 13a and 16a are arranged in positions rotated about the center of the orifice 12a of the nozzle member 12 by a certain angle.

The performance of the highest priority to the container-side aerosol forming member 13 is that the liquid 2 ejected in an aerosol form humidifies the oxygen gas, and a mixture of the air, the oxygen gas, and the aerosol is sent out from the horizontal projection portion 6 to the patient. In addition, the container-side suction passage 13b for sucking up the liquid 2, connected to the ejection port 13a, extends to near the inner bottom of the water bottle 1 at the lower end and is considerably long. The container-side aerosol forming member 13 therefore needs a somewhat large negative pressure to suck up the liquid 2 from the lower part of the water bottle 1.

On the other hand, the role of the receptor-side aerosol forming member 16 is to form the liquid 2 accumulated as droplets in the liquid receptor 15a in the lower part of the housing 15 of the nebulizer adaptor XB1, without being guided to the outside, into an aerosol again and send out the aerosol to the patient in combination with the liquid 2 ejected in an aerosol form from the container-side aerosol forming member 13. The priority of the role of the receptor-side aerosol forming member 16 is somewhat lower than that of the function of the container-side aerosol forming member 13. In addition, the receptor-side suction passage 16b of the receptor-side aerosol forming member 16 extends to near the bottom of the liquid receptor 15a. The distance from the liquid suction port 16c arranged at the lower end to the ejection port 16a of the liquid 2 is small. The negative pressure for sucking up the liquid 2 therefore may be smaller than the negative pressure needed for the container-side aerosol forming member 13.

Then, the ejection port 13a of the liquid 2 of the container-side aerosol forming member 13 is arranged in a position where the negative pressure is relatively large in the graphs of the negative pressure shown in FIGs. 4 and 5. The ejection port 16a of water of the receptor-side aerosol forming member 16 is arranged in a position where the negative pressure is relatively small. Such an arrangement makes sense.

FIGs. 3(A), 3(B), and 3(C) show specific examples thereof. It will be understood that these three examples are not restrictive. Other configurations may be employed as long as the arrangement satisfies the condition about the negative pressures needed for the ejection port 13a of the liquid 2 of the container-side aerosol forming member 13 and the ejection portion 16a of the liquid 2 of the receptor-side aerosol forming member 16.

The nebulizer XA1 described above includes the container-side aerosol forming member 13 configured to suck the liquid 2 from the water bottle 1 containing the liquid 2 for humidification and form an aerosol of the sucked liquid 2 by the negative pressure produced by the oxygen gas jetted from the orifice 12a of the nozzle member 12, the liquid receptor 15a configured to store the liquid 2 residing in the form of droplets into which the aerosol turns, and the receptor-side aerosol forming member 16 configured to suck the liquid 2 from the liquid receptor 15a and form the sucked liquid 2 into an aerosol again by the negative pressure. A drain tube for returning the liquid 2, such as sterile water, accumulated in the nebulizer XA1 to the container, such as the water bottle 1, therefore does not need to be provided. This facilitates the replacement of the container, such as the water bottle 1, and solves the conventional problem that the liquid 2 drips from the drain tube when the container is replaced.

The nebulizer XA1 does not need to include a drain tube for returning the liquid 2, such as sterile water, accumulated in the nebulizer adaptor XB1 to the container, such as the water bottle 1. Since the liquid 2, such as sterile water, to be a drain containing germs in the room will not return to the container, such as the water bottle 1, the drawback that the germs can get into the liquid in the container is solved.

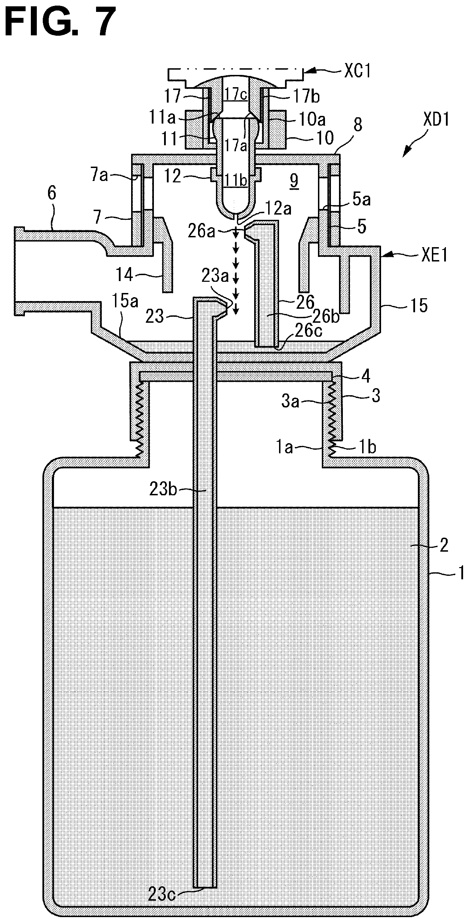

[Second Embodiment] Next, a configuration of a nebulizer XD1 according to a second embodiment will be described with reference to FIG. 7. FIG. 7 is a cross-sectional view for describing the configuration of the nebulizer XD1 according to the second embodiment of the present invention. Here, characteristic parts of the nebulizer XD1 will be mainly described. The same configuration and operation as those of the foregoing first embodiment will be denoted by the same reference numerals in the drawings, and a description thereof may be omitted as appropriate.

The nebulizer (humidifying device) XD1 shown in FIG. 7 includes a nebulizer adaptor XE1 and the water bottle (container) 1.

An ejection port 26a of an aerosol forming member 26 is arranged in a position to be affected by the oxygen gas jetted from the orifice 12a of the nozzle member 12. Since the nozzle member 12 and the aerosol forming member 26 have different functions, the members may be configured as two different members and may be combined with each other.

However, the positional relationship between the oxygen gas jetted from the orifice 12a of the nozzle member 12 and the ejection port 26a for jetting the liquid 2 is subtle and difficult to adjust. Therefore, the nozzle member 12 and the aerosol forming member 26 are preferably integrally configured.

A container-side suction member 23 is arranged below the diffuser 14 which is arranged in the housing 15 of the nebulizer adaptor XE1. An outlet 23a of the liquid 2 is formed in the extremity of the container-side suction member 23. A passage 23b for sucking up the liquid 2 is formed to be continued from the outlet 23a. The lower end of the passage 23b extends to near the inner bottom of the water bottle 1. The lower end is inserted in the liquid 2 such as sterile water, and can efficiently suck up the liquid 2 such as sterile water. In the present invention, a concept including both the ejection port (mainly intended for the formation of an aerosol) described in the first embodiment and the foregoing outlet (not mainly intended for the formation of an aerosol) in the second embodiment is defined as a discharge port. This container-side suction member 23 serves as a suction mechanism configured to suck the liquid 2 from the water bottle 1 by the negative pressure produced by the gas.

The liquid 2 is discharged from the outlet 23a of the container-side suction member 23 by the negative pressure produced by the oxygen gas jetted from the orifice 12a of the nozzle member 12. The liquid receptor 15a can store the liquid 2.

The aerosol forming member 26 is formed inside the diffuser 14 which is arranged in the housing 15 of the nebulizer adaptor XE1. The ejection port 26a of the liquid 2 of the aerosol forming member 26 is formed near the orifice 12a of the nozzle member 12. The lower end of the aerosol forming member 26 extends to near the bottom of the foregoing liquid receptor 15a, where a liquid suction port 26c is arranged.

The liquid 2 that is discharged from the outlet 23a of the container-side suction member 23 and accumulated in the liquid receptor 15a in the lower part of the housing 15 of the nebulizer adaptor XE1 is sucked from the liquid suction port 26c. The liquid 2 passes through a receptor-side suction passage 26b and is ejected from the ejection port 26a of the liquid 2 of the receptor-side aerosol forming member 26 in the form of aerosol. The resulting mixture of the air, the oxygen gas, and the aerosol is sent out from the horizontal projection portion 6 to the patient.

The positional relationship between the outlet 23a of the liquid 2 of the container-side suction member 23 and the ejection port 26a of the liquid 2 of the aerosol forming member 26 is such that the container-side suction member 23 is placed in a position to not interfere with the formation of the aerosol and the humidification of the oxygen gas by the liquid 2 from the ejection port 26a of the liquid 2 of the aerosol forming member 26.

The nebulizer XD1 described above includes the container-side suction member 23 configured to suck the liquid 2 from the water bottle 1 containing the liquid 2 for humidification by the negative pressure produced by the oxygen gas jetted from the orifice 12a of the nozzle member 12, the liquid receptor 15a configured to store the liquid 2 sucked by the container-side suction member 23, and the aerosol forming member 26 configured to suck the liquid 2 from the liquid receptor 15a and form an aerosol of the sucked liquid 2 by the negative pressure. A drain tube for returning the liquid 2, such as sterile water, accumulated in the nebulizer XD1 to the container, such as the water bottle 1, therefore does not need to be provided. This facilitates the replacement of the container, and solves the conventional problem that the liquid 2 drips from the drain tube when the container is replaced.

The nebulizer XD1 does not need to include a drain tube for returning the liquid 2, such as sterile water, accumulated in the nebulizer adaptor XE1 to the container, such as the water bottle 1. Since the liquid 2, such as sterile water, to be a drain containing germs in the room will not return to the container, such as the water bottle 1, the drawback that the germs can get into the liquid 2 in the container is solved.

In the nebulizer XD1, if the container-side suction member 23 continues sucking in the liquid 2 in the water bottle 1 and the liquid surface of the liquid 2 stored in the liquid receptor 15a rises, the outlet 23a of the container-side suction member 23 is submerged in the liquid 2. This stops the function of sucking up the liquid 2 by the container-side suction member 23. The liquid 2 accumulated in the liquid receptor 15a is thereby prevented from overflowing into the horizontal projection portion 6. As the aerosol forming member 26 sucks up the liquid 2 stored in the liquid receptor 15a, the liquid surface of the liquid 2 falls and the outlet 23a of the container-side suction member 23 emerges from the liquid surface of the liquid 2. The function of sucking up the liquid 2 by the container-side suction member 23 is thereby restored.

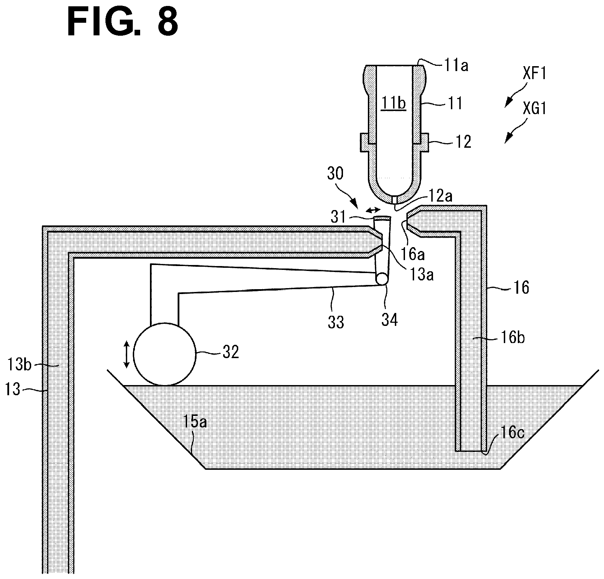

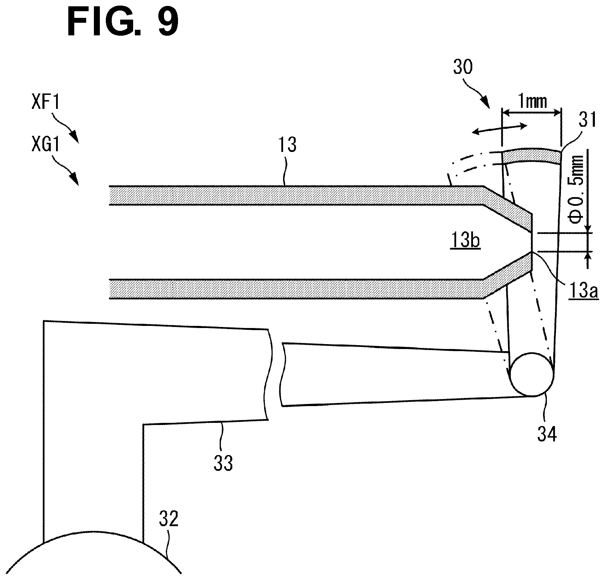

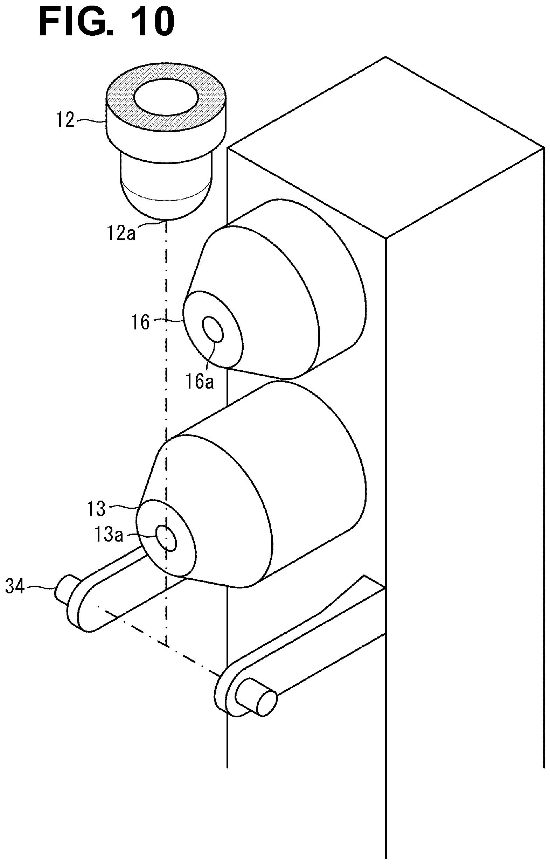

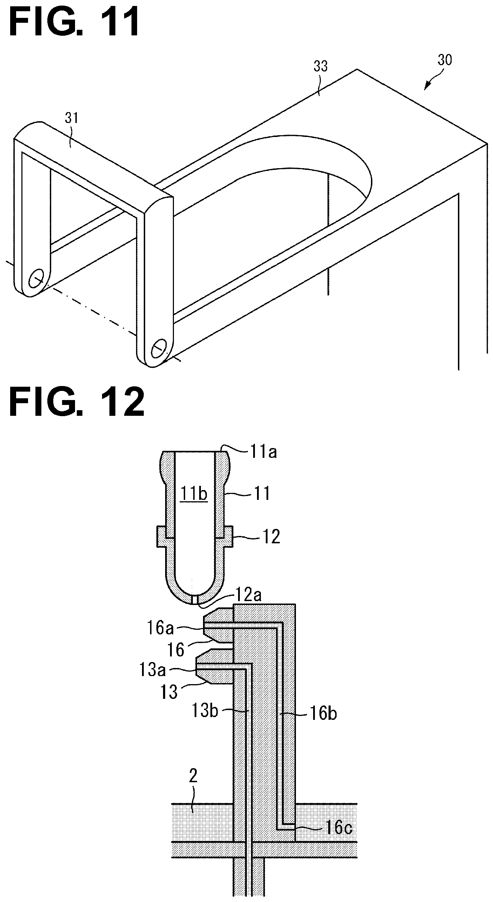

[Third Embodiment] Next, a configuration of a nebulizer XF1 according to a third embodiment will be described with reference to FIGs. 8 to 12. FIG. 8 is a conceptual diagram for describing a structure of a valve 30 included in a nebulizer adaptor XG1 which constitutes the nebulizer XF1. FIG. 9 is an enlarged view for describing the structure of the valve 30. FIG. 10 is a perspective view showing a positional relationship between an ejection port 13a of a container-side aerosol forming member 13, an ejection port 16a of a receptor-side aerosol forming member 16, and an orifice 12a of a nozzle member 12. FIG. 11 is a perspective view of the valve 30. FIG. 12 is a cross-sectional view showing a positional relationship between the ejection port 13a of the container-side aerosol forming member 13, the ejection port 16a of the receptor-side aerosol forming member 16, and the orifice 12a of the nozzle member 12. Here, characteristic parts of the nebulizer adaptor XG1 will be mainly described. The same configuration and operation as those of the foregoing embodiments will be denoted by the same reference numerals in the drawings, and a description thereof may be omitted as appropriate.

The nebulizer (humidifying device) XF1 shown in FIGs. 8 and 9 includes the nebulizer adaptor XG1 and a not-shown water bottle (container).

As shown in FIG. 8, the ejection port 13a of the container-side aerosol forming member 13 and the ejection port 16a of the receptor-side aerosol forming member 16 are opposed to each other and are both arranged along a flow of a gas jetted from the orifice 12a in series. Specifically, the ejection port 16a of the receptor-side aerosol forming member 16 is close to the orifice 12a of the nozzle member 12. The ejection port 13a of the container-side aerosol forming member 13 is located in a position farther from the orifice 12a than the ejection port 16a is. As shown in FIG. 12, the ejection port 13a of the container-side aerosol forming member 13 and the ejection port 16a of the receptor-side aerosol forming member 16 may be arranged in the same direction.

The nebulizer adaptor XG1 includes a suction force control mechanism configured to make the capability of the suction mechanism (container-side aerosol forming member 13) to suck the liquid 2 variable by using a rise or fall of the liquid surface of the liquid 2 stored in the liquid receptor 15a. As the suction force control mechanism, the present embodiment includes the valve 30 configured to open and close the ejection port 13a of the container-side aerosol forming member 13. The valve 30 includes a cutoff member 31, a floating member 32, and a connection member 33.

The cutoff member 31 oscillates about an oscillation shaft 34 between a cutoff position (position shown by the solid lines in FIG. 9) in which to cut off the ejection port 13a of the container-side aerosol forming member 13 from the orifice 12a of the nozzle member 12 and a retracted position (position shown by the dashed-dotted lines in FIG. 9) in which to be retracted from the cutoff position. If the cutoff member 31 is located in the cutoff position, the cutoff member 31 cuts off the ejection port 13a of the container-side aerosol forming member 13 from the flow of the gas jetted from the orifice 12 of the nozzle member 12 and thereby stops the function of sucking up the liquid 2 by the container-side aerosol forming member 13. If the cutoff member 31 is located in the retracted position, the cutoff member 31 cancels the cutoff of the ejection port 13a from the flow of the gas jetted from the orifice 12a and thereby restores the function of sucking up the liquid 2 by the container-side aerosol forming member 13.

The floating member 32 floats on the liquid surface of the liquid 2 stored in the liquid receptor 15a, and floats up and down according to the rise or fall of the liquid surface. The connection member 33 connects the floating member 32 to the cutoff member 31 with the oscillation shaft 34. The connection member 33 makes the cutoff member 31 to oscillate with the floating of the floating member 32.

In such a nebulizer XF1, if the container-side aerosol forming member 13 continues sucking up the liquid 2 in the water bottle and the liquid surface of the liquid 2 stored in the liquid receptor 15a rises, the floating member 32 floats up. The cutoff member 31 oscillates to the cutoff position, and the function of sucking up the liquid 2 by the container-side aerosol forming member 13 stops. The liquid 2 accumulated in the liquid receptor 15a is thereby prevented from overflowing into the horizontal projection portion 6 (see FIG. 1). As the receptor-side aerosol forming member 16 sucks up the liquid 2 stored in the liquid receptor 15a, the liquid surface of the liquid 2 falls. The floating member 32 floats down, the cutoff member 31 oscillates to the retracted position, and the function of sucking up the liquid 2 by the container-side aerosol forming member 13 is restored. Such a system has an advantage that the pressure of the sprayed oxygen gas is received by the oscillation shaft 34, and thus, the rotation of the cutoff member 31 by the buoyancy of the floating member 32 according to a change in the liquid level of the liquid 2 is less likely to be adversely affected.

As shown in FIGs. 10 to 12, the container-side aerosol forming member 13, the receptor-side aerosol forming member 16, and the oscillation shaft 34 are preferably integrally configured.

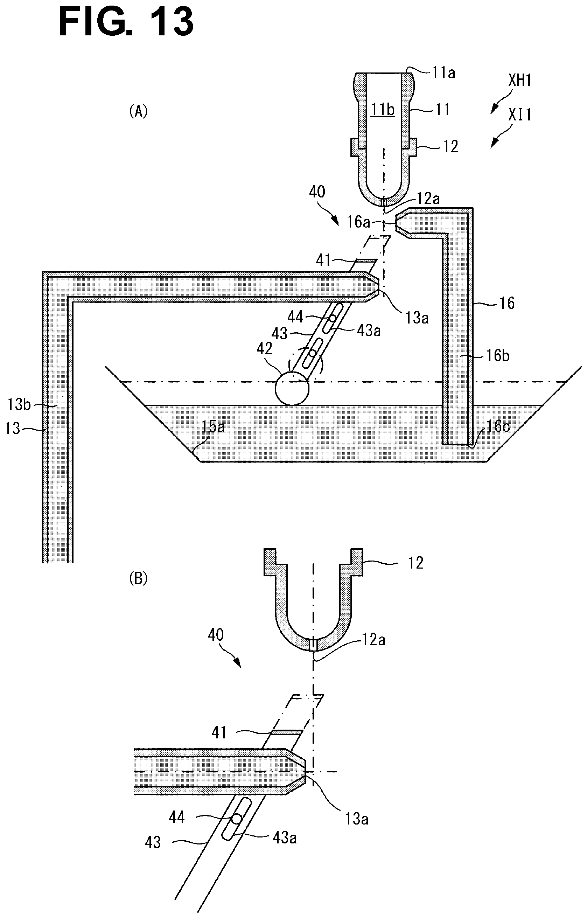

[Fourth Embodiment] Next, a configuration of a nebulizer XH1 according to a fourth embodiment will be described with reference to FIG. 13. FIG. 13(A) is a conceptual diagram for describing a structure of a valve 40 included in a nebulizer adaptor XI1 which constitutes the nebulizer XH1 according to the fourth embodiment of the present invention. FIG. 13(B) is an enlarged view showing essential parts of the structure shown in FIG. 13(A).

The nebulizer (humidifying device) XH1 shown in FIGs. 13(A) and 13(B) includes the nebulizer adaptor XI1 and a not-shown water bottle (container).

The nebulizer adaptor XI1 includes the valve 40 configured to cut off the ejection port 13a of the container-side aerosol forming member 13 from the flow of the gas jetted from the orifice 12a. The valve 40 is of an "oblique slide system" in which the flow of the oxygen gas itself jetted from the orifice 12a is cut off in front of the ejection port 13a. Specifically, the valve 40 includes a cutoff member 41, a floating member 42, and a connection member 43.

The cutoff member 41 makes an oblique sliding movement between a cutoff position (position shown by the dashed-dotted lines in FIG. 13(A)) in which to cut off the ejection port 13a from the orifice 12a and a retracted position (position shown by the solid lines in FIG. 13(A)) in which to be retracted from the cutoff position. If the cutoff member 41 is located in the cutoff position, the cutoff member 41 cuts off the ejection port 13a from the orifice 12a to stop the function of sucking up the liquid 2 by the container-side aerosol forming member 13. If the cutoff member 41 is located in the retracted position, the cutoff member 41 cancels the cutoff of the ejection port 13a from the orifice 12a to restore the function of sucking up the liquid 2 by the container-side aerosol forming member 13.

The floating member 42 floats on the liquid surface of the liquid 2 stored in the liquid receptor 15a, and floats up and down according to the rise or fall of the liquid surface. Guide grooves 43a to be guided by guide pins 44 formed on the housing (not shown) of the nebulizer adaptor XI1 are formed in the connection member 43. This connection member 43 connects the floating member 42 to the cutoff member 41, and causes the cutoff member 41 to make a sliding movement with the floating of the floating member 42.

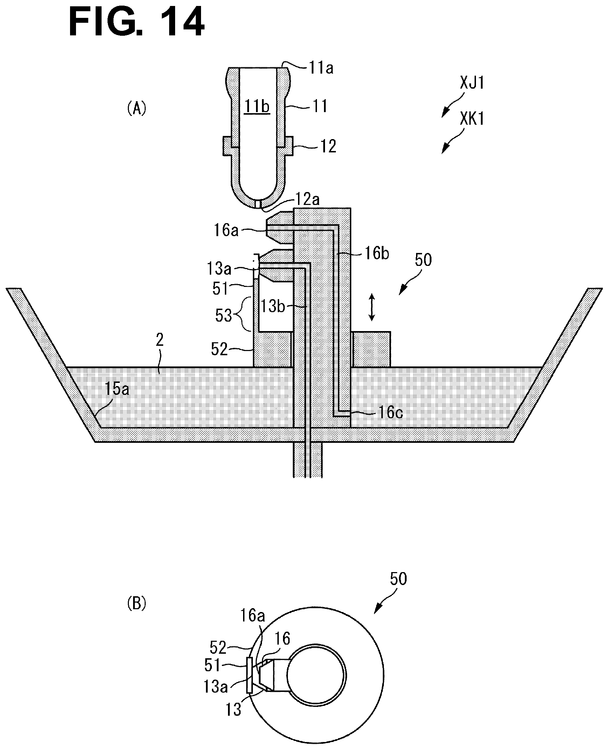

[Fifth Embodiment] Next, a configuration of a nebulizer XJ1 according to a fifth embodiment will be described with reference to FIG. 14. FIG. 14(A) is a conceptual diagram for describing a structure of a valve 50 included in a nebulizer adaptor XK1 which constitutes the nebulizer XJ1 according to the fifth embodiment of the present invention. FIG. 14(B) is a top view showing essential parts of the structure shown in FIG. 14(A).

The nebulizer (humidifying device) XJ1 shown in FIGs. 14(A) and 14(B) includes the nebulizer adaptor XK1 and a not-shown water bottle (container).

The nebulizer adaptor XK1 includes the valve 50 configured to open and close the ejection port 13a of the container-side aerosol forming member 13. The valve 50 is of a "vertical slide system" for blocking the ejection port 13a. Specifically, the valve 50 includes a cutoff plate 51, a float ring 52 which is a floating member, and a connection member 53 configured to connect the cutoff plate 51 and the float ring 52.

The cutoff plate 51 is integrally configured with the float ring 52 and makes a sliding movement with the float ring 52. The cutoff plate 51 thus makes a vertical sliding movement between a cutoff position (position shown by the dashed-dotted lines in FIG. 14(A)) in which to block the ejection port 13a and a retracted position (position shown by the solid lines in FIG. 14(A)) in which to be retracted from the cutoff position. If the cutoff plate 51 is located in the cutoff position, the cutoff plate 51 blocks the ejection port 13a to stop the function of sucking up the liquid 2 by the container-side aerosol forming member 13. If the cutoff plate 51 is located in the retracted position, the cutoff plate 51 cancels the blocking of the ejection port 13a to restore the function of sucking up the liquid 2 by the container-side aerosol forming member 13.

The float ring 52 is slidably fitted onto the container-side aerosol forming member 13 and the receptor-side aerosol forming member 16 which are integrally configured. The float ring 52 floats on the liquid surface of the liquid 2 stored in the liquid receptor 15a, and floats up and down according to the rise or fall of the liquid surface. The float ring 52 thereby causes the cutoff plate 51 to make a vertical sliding movement.

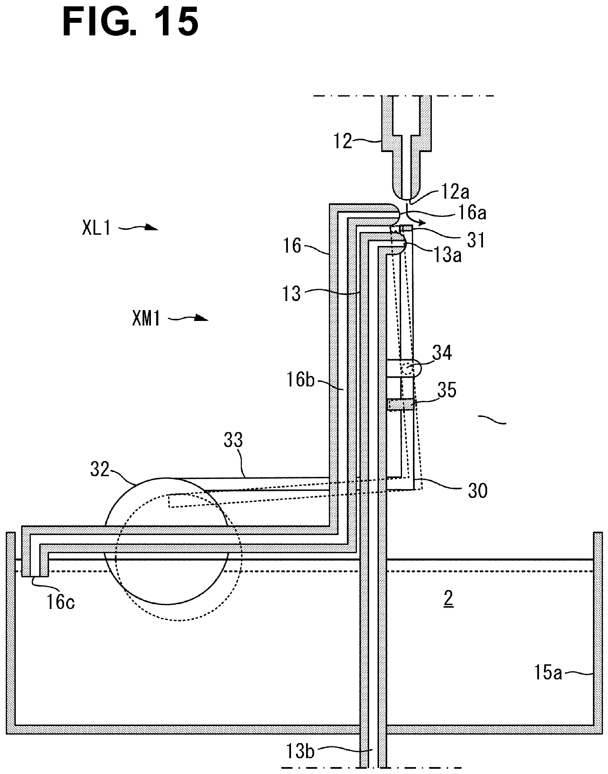

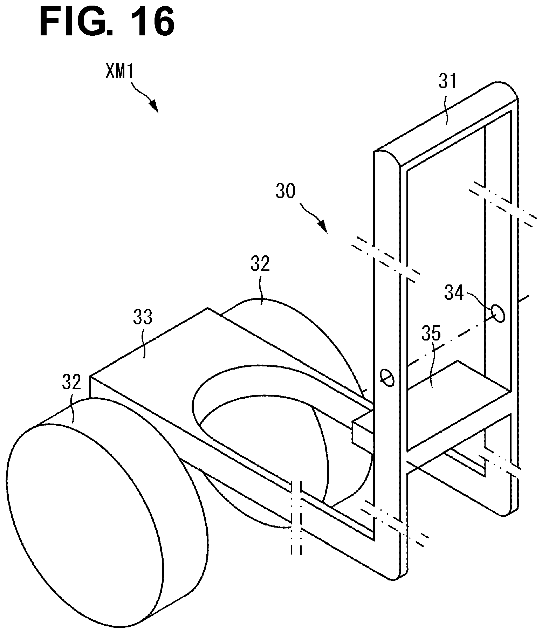

[Sixth Embodiment] Next, a configuration of a nebulizer XL1 according to a sixth embodiment will be described with reference to FIGs. 15 and 16. This is a modification of the third embodiment. FIG. 15 is a conceptual diagram for describing a structure of a valve 30 (suction force control mechanism) included in a nebulizer adaptor XM1 which constitutes the nebulizer XL1 according to the sixth embodiment of the present invention. FIG. 16 is a perspective view of the valve 30. Here, characteristic parts of the nebulizer adaptor XM1 will be mainly described. The same configuration and operation as those of the foregoing embodiments will be denoted by the same reference numerals in the drawings, and a description thereof may be omitted as appropriate.

The suction force control mechanism of the nebulizer adaptor XM1 also has a function of maintaining the liquid surface of the liquid 2 stored in the liquid receptor 15a in a prescribed position. Specifically, if the liquid surface of the liquid 2 in the liquid receptor 15a reaches the prescribed position, the suction force control mechanism reduces the suction capability of the container-side aerosol forming member 13. On the other hand, if the liquid surface of the liquid 2 in the liquid receptor 15a falls below the prescribed position, the suction force control mechanism increases the suction capability of the container-side aerosol forming member 13. Specifically, the suction force control mechanism includes the valve 30 configured to make an opening and closing operation vertically above the ejection port 13a and thereby prevent the oxygen jetted from the orifice 12a from impinging on the ejection port 13a of the container-side aerosol forming member 13. As shown in FIG. 15, the valve 30 includes a cutoff member 31, floating members 32, a connection member 33, and a stopper 35.

As shown in FIG. 16, the stopper 35 is arranged on a connection member 33. When the cutoff member 31 oscillates in the cutoff direction, the stopper 35 comes into contact with a side surface of the container-side aerosol forming member 13 to position the cutoff member 31. If the cutoff member 31 oscillates in the retracting direction, the cutoff member 31 itself comes into contact with the container-side aerosol forming member 13 to position itself. Consequently, the oscillation range of the cutoff member 31 can be regulated by both the stopper 35 and container-side aerosol forming member 13 for more precise liquid surface control.

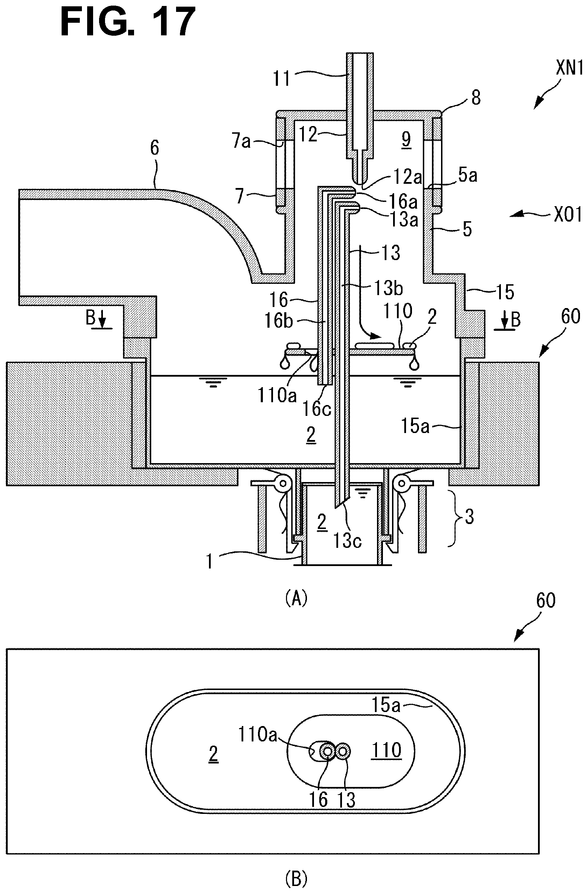

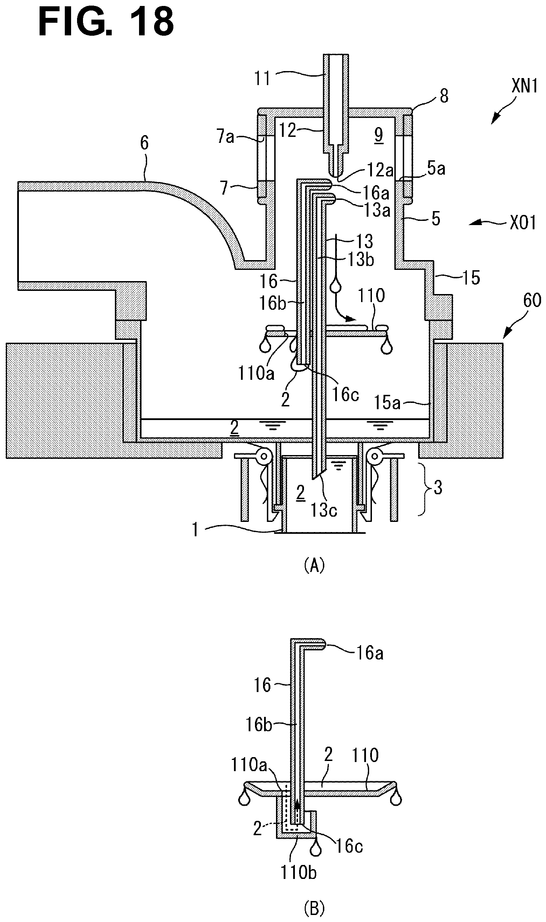

[Seventh Embodiment] Next, a configuration of a nebulizer XN1 according to a seventh embodiment will be described with reference to FIGs. 17 and 18. Here, characteristic parts of the nebulizer XN1 will be mainly described. The same configuration and operation as those of the foregoing first embodiment will be designated by the same reference numerals in the drawings, and a description thereof may be omitted as appropriate. To avoid complication of the drawings, a description and depiction of the suction force control mechanism are omitted.

This nebulizer (humidifying device) XN1 includes a nebulizer adaptor XO1, a heater device 60, and a water bottle (container) 1.

The heater device 60 heats at least the liquid 2 in the liquid receptor 15a from outside. The liquid suction port 16c of the receptor-side aerosol forming member 16 preferentially sucks part of the liquid 2 near the surface layer (liquid surface) in the liquid receptor 15a. Since the high-temperature liquid 2 heated by the heater device 60 resides near the liquid surface, such a liquid 2 can be preferentially sucked to quickly increase the humidifying temperature.

The nebulizer adaptor XO1 further includes a second liquid receptor 110 capable of receiving the liquid 2 aside from the liquid receptor 15a, vertically beneath the ejection port 16a and above the liquid surface in the liquid receptor 15a. The second liquid receptor 110 is a plate member that is fixed to the receptor-side aerosol forming member 16 or the container-side aerosol forming member 13 and extends in a horizontal direction (or oblique direction). The second liquid receptor 110 is not able to receive a large amount of liquid 2 but can preferentially retain the liquid 2 dripping from the container-side aerosol forming member 13 on the surface. The second liquid receptor 110 has a notch (or opening) 110a near the receptor-side aerosol forming member 16. The second liquid receptor 110 can let the retained liquid 2 flow down along the peripheral wall of the receptor-side aerosol forming member 16 via the notch 110a.

As shown in FIG. 17, if a sufficient amount of the liquid 2 is accumulated in the liquid receptor 15a, the liquid flowing down along the peripheral wall of the receptor-side aerosol forming member 16 is supplied to the liquid receptor 15a.

On the other hand, as shown in FIG. 18(A), in an initial state where the liquid 2 is not accumulated in the liquid receptor 15a or if the liquid level of the liquid receptor 15a falls, the liquid flowing down from the second liquid receptor 110 through the notch (or opening) 110a along the peripheral wall of the receptor-side aerosol forming member 16 is all sucked from the liquid suction port 16c and ejected from the ejection port 16a as an aerosol. That is, the present embodiment can quickly perform humidification even before the liquid 2 is accumulated in the liquid receptor 15a. This is particularly effective if the liquid suction port 16c is desired to be positioned near the liquid surface of the liquid receptor 15a at full liquid time in view of influence of the heating by the heater device 60 and the like.

The second liquid receptor 110 also serves as a restriction member configured to restrict the impingement of the gas jetted from the orifice 12a of the nozzle member 12 on the liquid surface of the liquid receptor 15a. This restriction member can receive the gas jetted from the orifice 12a to suppress rippling of the liquid surface of the liquid receptor 15a due to the jetting of the gas. Since the second liquid receptor 110 functions as the restriction member, the heated liquid 2 in the liquid receptor 15a heated by the heater device 60 can reside still near the liquid surface. Only the warm liquid can thus be stably and efficiently sucked from the liquid suction port 16c of the receptor-side aerosol forming member 16.

While the second liquid receptor 110 here is described to a plate member, the second liquid receptor 110 may be configured in a dish-like or container-like shape as shown in FIG. 18(B) so that a larger amount of the liquid 2 can be stored. A guide channel 110b configured to guide the liquid 2 accumulated in the second liquid receptor 110 to the liquid suction port 16c of the receptor-side aerosol forming member 16 may be provided.

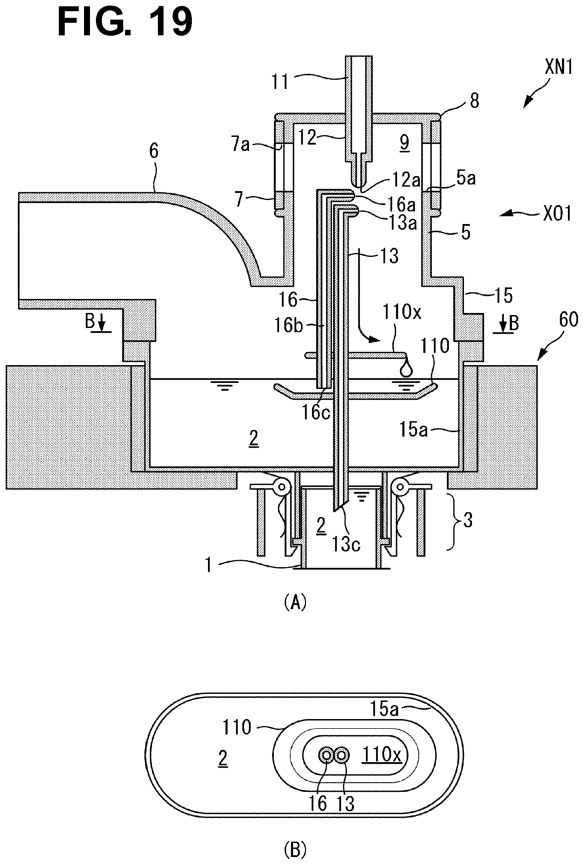

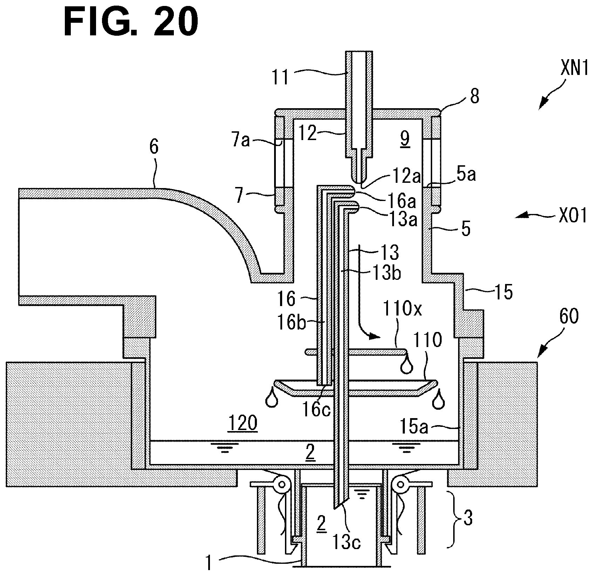

While the second liquid receptor 110 is described to be arranged above the liquid surface (at full liquid time) of the liquid receptor 15a, the present invention is not limited thereto. For example, as shown in FIGs. 19 and 20, the second liquid receptor 110 may be arranged below the liquid surface (at full liquid time). In such a case, the liquid suction port 16c of the receptor-side aerosol forming member 16 is preferably arranged so that the liquid accumulated in the second liquid receptor 110 can be directly sucked. With such a configuration, as shown in FIG. 19, if the liquid receptor 15a is full of the liquid, the second liquid receptor 110 is submerged in the liquid and provides no function. As shown in FIG. 20, in an initial state where the liquid 2 is not accumulated in the liquid receptor 15a or if the liquid level of the liquid receptor 15a falls for some reason, the second liquid receptor 110 emerges and preferentially retains the liquid 2 dripping from the container-side aerosol forming member 13 so that the liquid 2 can be quickly supplied to the receptor-side aerosol forming member 16. While FIGs. 19 and 20 show a case where a restriction member 110x is arranged aside from the second liquid receptor 110, the restriction member 110x may be omitted.

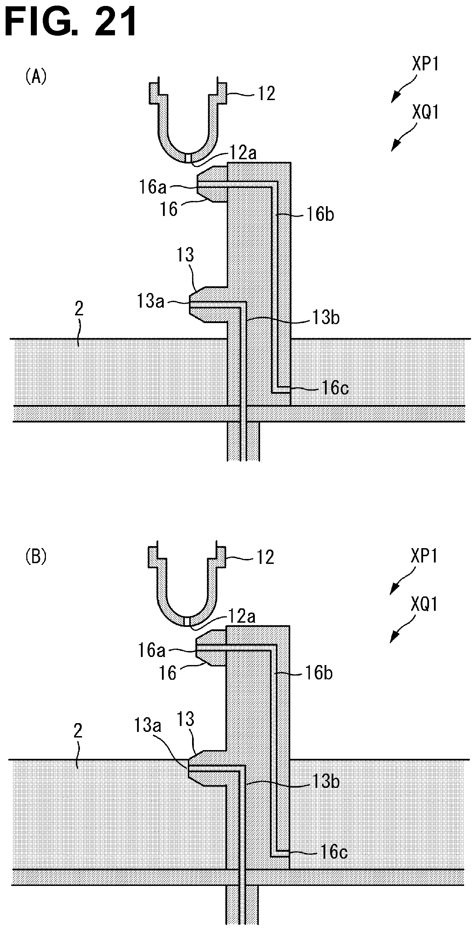

[Eighth Embodiment] Next, a configuration of a nebulizer XP1 according to an eighth embodiment will be described with reference to FIG. 21. The nebulizer (humidifying device) XP1 shown in FIGs. 21(A) and 21(B) includes a nebulizer adaptor XQ1, and a heater device and a water bottle (container) which are not shown in the diagrams.

In the nebulizer adaptor XQ1, the ejection port 13a of the container-side aerosol forming member 13 is arranged as a suction force control mechanism, at approximately the same height as the prescribed liquid level. In other words, the nebulizer adaptor XQ1 is of "submersion type" in which the ejection port 13a is blocked by the liquid surface of the liquid 2.

In the state shown in FIG. 21(A), the liquid surface of the liquid 2 is lower than the ejection port 13a. The exposed ejection port 13a enables the sucking up of the liquid 2 in the water bottle and the ejection of the liquid 2 as an aerosol. On the other hand, in the state shown in FIG. 21(B) where the liquid level of the liquid 2 rises and the ejection port 13a is submerged, the function of sucking up the liquid 2 by the container-side aerosol forming member 13 stops. If the liquid 2 is consumed as an aerosol and the liquid surface falls, the state returns to that of FIG. 21(A) again. The ejection port 13a is exposed to restore the function of sucking up the liquid 2 by the container-side aerosol forming member 13.

In the present embodiment, the ejection port 13a of the container-side aerosol forming member 13 is located closer to the liquid surface side. The ejection port 13a is thus farther from the orifice 12a, and the negative pressure may become insufficient. In such a case, the flow of the oxygen gas may be branched to form a second orifice aside from the orifice 12a corresponding to the ejection port 16a of the receptor-side aerosol forming member 16. The second orifice and the ejection port 13a of the container-side aerosol forming member 13 arranged near the prescribed liquid level may be located close to each other.

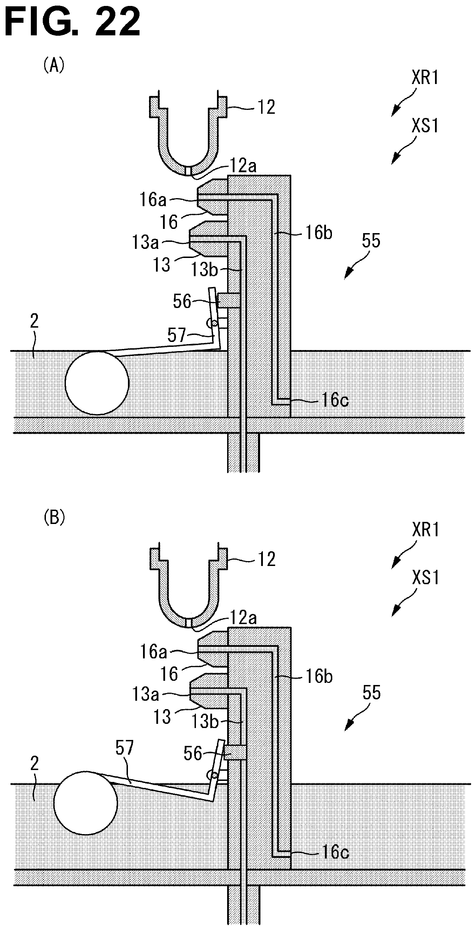

[Ninth Embodiment] Next, a configuration of a nebulizer XR1 according to a ninth embodiment will be described with reference to FIG. 22. The nebulizer (humidifying device) XR1 shown in FIGs. 22(A) and 22(B) includes a nebulizer adaptor XS1, and a heater device and a water bottle (container) which are not shown in the diagrams.

The nebulizer adaptor XS1 includes, as a suction force control mechanism, a valve 56 which is arranged on the way of the container-side suction passage 13b of the container-side aerosol forming member 13, and a driving member 57 configured to oscillate according to the rise or fall of the liquid 2 by using buoyancy and switch ON/OFF the valve 56. The valve 56 moves toward and away from the container-side suction passage 13b to close and open the flow channel of the container-side suction passage 13b. As shown in FIG. 22(A), if the liquid level of the liquid 2 is low, the valve 56 opens the container-side suction passage 13b so that the container-side aerosol forming member 13 can suck up the liquid 2. On the other hand, as shown in FIG. 22(B), if the liquid surface of the liquid 2 reaches a prescribed position, the driving member 57 oscillates and pushes in the valve 56 to close the container-side suction passage 13b. As a result, the function of sucking up the liquid 2 by the container-side aerosol forming member 13 stops.

In such embodiments, the suction force control mechanism is described to switch the sucking function of the container-side aerosol forming member 13 by using the buoyancy of the liquid 2 or the liquid itself. However, the present invention is not limited thereto. For example, the liquid surface (liquid level) of the liquid 2 may be electrically measured, and the measurement result may be used to switch ON/OFF the flow of the container-side suction passage 13b by an electrical valve. If the liquid is sucked up from the water bottle by an electrical pump, the pump function may be switched ON/OFF to control the liquid surface by utilizing measurement results of the liquid surface.

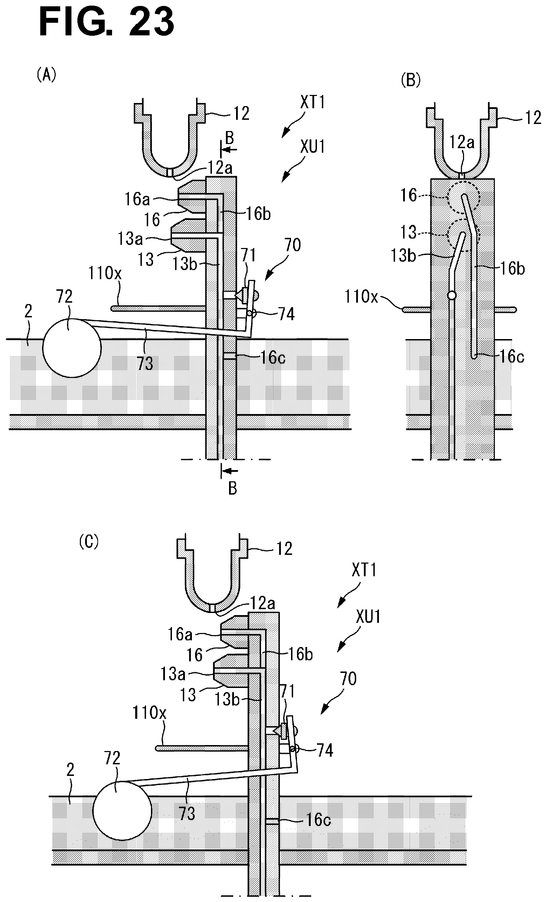

[Tenth Embodiment] Next, a configuration of a nebulizer XT1 according to a tenth embodiment will be described with reference to FIGs. 23 to 25. The nebulizer (humidifying device) XT1 shown in FIGs. 23(A) to 23(C) includes a nebulizer adaptor XU1, and a heater device and a water bottle (container) which are not shown in the diagrams.

As shown in FIG. 23(B), the container-side suction passage 13b of the container-side aerosol forming member 13 and the receptor-side suction passage 16b of the receptor-side aerosol forming member 16 are arranged in parallel in a circumferential direction with the flowing direction of the gas jetted from the orifice 12a as the main axis.

The suction force control mechanism includes an air opening port 13d which is formed in the middle of the container-side suction passage 13b, above the liquid level of the liquid receptor 15a at full liquid time, and an air control valve 70 configured to open and close the air opening port 13d. The air opening port 13d is an opening having a circular cross section, perpendicularly intersecting with the container-side suction passage 13b. The air opening port 13d makes the container-side suction passage 13b communicate with the air.

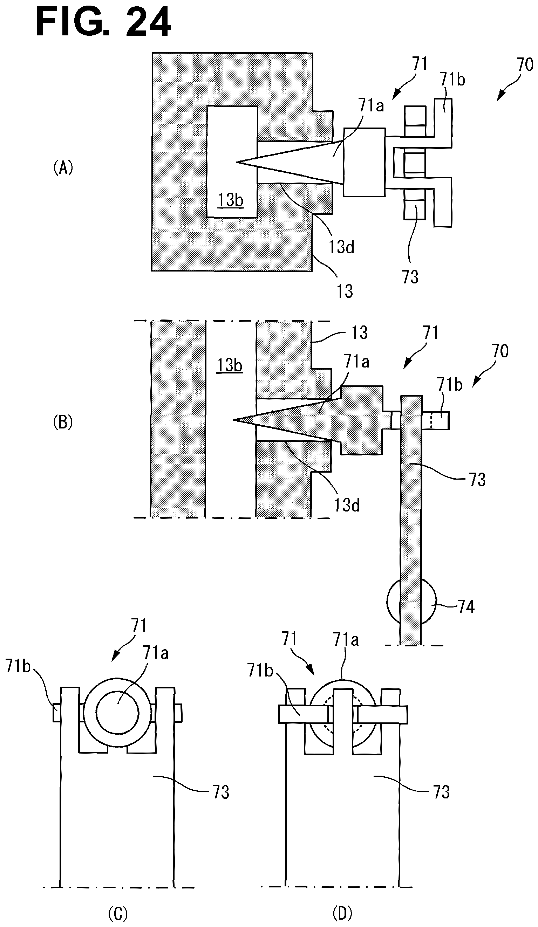

The air control valve 70 includes an opening and closing member 71, a floating member 72, and a connection member 73. As shown in FIG. 24, the opening and closing member 71 includes a valve body 71a of conical shape which is inserted into the air opening port 13a with its pointed side first, and an engagement portion 71b which is formed on a rear side of the valve body 71a. The floating member 72 floats on the liquid surface of the liquid 2 stored in the liquid receptor 15a, and floats up and down according to the rise or fall of the liquid surface. The connection member 73 is connected at one end to the floating member 72, and engaged at the other end with the engagement portion 71b of the opening and closing member 71. The connection member 73 can oscillate freely on an oscillation shaft 74. If the connection member 73 oscillates according to the floating of the floating member 72, the valve body 71a of the engaged opening and closing member 71 moves toward and away from the air opening port 13d. The reasons why the valve body 71a has the conical shape are that if the valve body 71a is inserted into the air opening port 13d of circular shape with its pointed side first, the two members are autonomously centered, that the flowrate can be fine adjusted by the degree of insertion, and that the sealability can be improved when the valve body 71a is fully inserted. When the valve body 71a is fully inserted, the flat surface around the bottom of the valve body 71a can be brought into close contact with the flat surface around the air opening port 13d to hermetically seal the air opening port 13d.

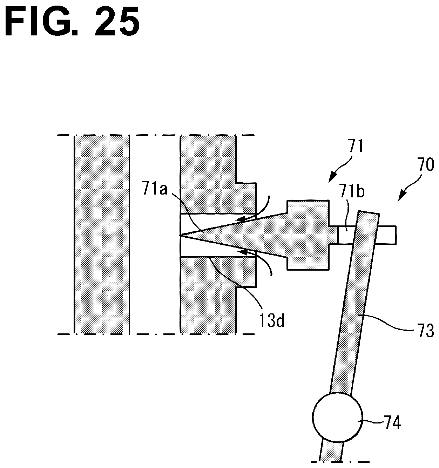

As shown in FIGs. 23(A) and 25, if the liquid level of the liquid 2 rises and the floating member 72 floats up, the valve body 71a therefore moves away from the air opening port 13d to let the air into the container-side suction passage 13b. This makes the container-side aerosol forming member 13 not able to suck the liquid in the water bottle (not shown). On the other hand, as shown in FIGs. 23(C) and 24, if the liquid level of the liquid 2 falls and the floating member 72 lowers, the valve body 71a moves into the air opening port 13d to block the air opening port 13d. As a result, the sealability of the container-side suction passage 13b increases, and the liquid 2 in the water bottle 1 can be sucked. In fact, the gap between the air opening port 13d and the valve body 71a autonomously comes to a balance at an optimum distance to maintain a state where the consumption of the liquid 2 by humidification and the amount of the liquid 2 sucked by the container-side aerosol forming member 13 equal to each other.

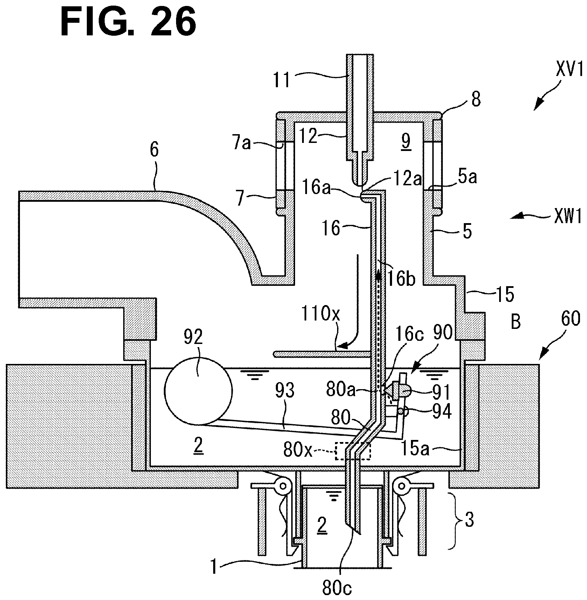

[Eleventh Embodiment] Next, a configuration of a nebulizer XV1 according to an eleventh embodiment will be described with reference to FIGs. 26 to 29. Here, characteristic parts of a nebulizer adaptor XW1 will be mainly described. The same configuration and operation as those of the foregoing embodiments will be designated by the same reference numerals, and a description thereof may be omitted as appropriate.

The nebulizer adaptor XW1 includes a receptor-side aerosol forming member 16. The receptor-side aerosol forming member 16 includes an ejection port 16a which is arranged near the orifice 12a of the nozzle member 12, a liquid suction port 16c configured to open in the liquid 2 of the liquid receptor 15a, and a receptor-side suction passage 16b configured to make the ejection port 16a and the liquid suction port 16c communicate with each other.

A communication passage 80 configured to communicate the liquid 2 in the water bottle 1 to the receptor-side suction passage 16b is further provided as a suction mechanism configured to suck the liquid 2 from the water bottle 1. The upper end of the communication passage 80 serves as a confluent port 80a to join the receptor-side suction passage 16b. The lower end of the communication passage 80 serves as a liquid suction port 80c configured to suck the liquid 2 of the water bottle 1. In the present embodiment, the receptor-side suction passage 16b and the communication passage 80 are connected in series, and the liquid suction port 16c having a circular (or cylindrical) cross section is formed in the side surface. If a negative pressure is applied to the ejection port 16a of the receptor-side aerosol forming member 16, the liquid 2 of the liquid receptor 15a is therefore attempted to be sucked from the liquid suction port 16c of the receptor-side suction passage 16b, and the liquid 2 in the water bottle 1 is attempted to be sucked from the liquid suction port 80c of the communication passage 80 at the same time. In other words, in such a structure, the suction force of the ejection port 16a of the receptor-side aerosol forming member 16 is used to suck the liquid 2 from the water bottle 1.

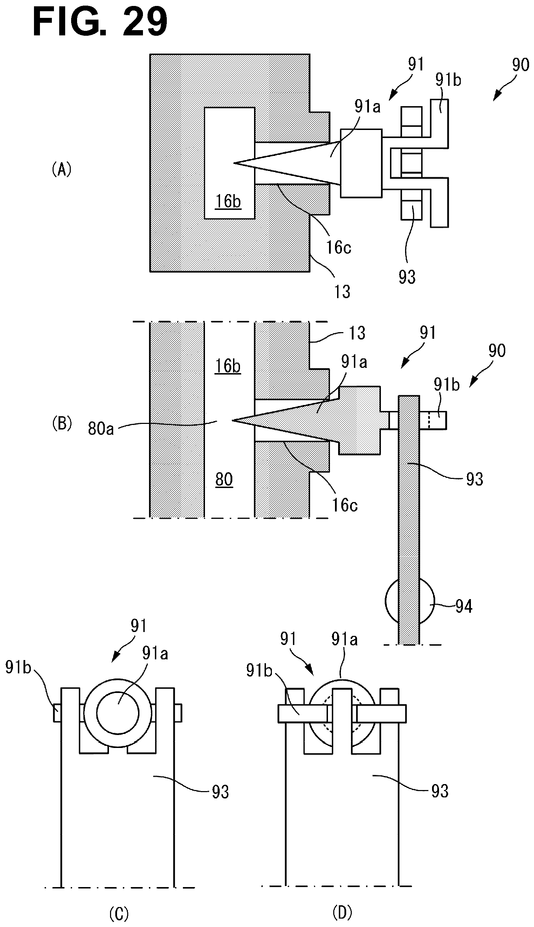

The present embodiment further includes a suction force control mechanism configured to make the capability of the suction mechanism to suck the liquid 2 of the water bottle 1 variable by using the rise or fall of the liquid surface of the liquid 2 stored in the liquid receptor 15a. Various configurations may be employed for such a suction force control mechanism. For example, as shown in FIG. 29, a control member 90 configured to open and close the liquid suction port 16c of the receptor-side suction passage 16b is preferably used to control the amount of the liquid 2 sucked from the liquid receptor 15a. If the suction amount is suppressed by the control member 90, the suction force (negative pressure) of the ejection port 16a is supplied to the communication passage 80 side. This can accelerate the suction of the liquid 2 of the water bottle 1.

Specifically, the control member 90 according to the present embodiment includes an opening and closing member 91 configured to move between a closing position for closing the liquid suction port 16c of the liquid 2 of the liquid receptor 15a and an opening position for opening the liquid suction port 16c, a floating member 92 configured to float up and down according to the rise or fall of the liquid surface of the liquid 2 stored in the liquid receptor 15a, and a connection member 93 configured to connect the floating member 92 and the opening and closing member 91 and make the opening and closing member 91 move with the floating of the floating member 92.

As shown in FIG. 29, the opening and closing member 91 includes a valve body 91a which has a conical shape and is inserted into the liquid suction port 16c with its pointed side first, and an engagement portion 91b which is formed on a rear side of the valve body 91a. The floating member 92 floats on the liquid surface of the liquid 2 stored in the liquid receptor 15a, and floats up and down according to the rise or fall of the liquid surface. The connection member 93 is connected at one end to the floating member 92, and engaged at the other end with the engagement portion 91b of the opening and closing member 91. The connection member 93 can oscillate freely on an oscillation shaft 94. If the connection member 93 oscillates according to the floating of the floating member 92, the valve body 91a of the engaged opening and closing member 91 moves toward or away from the liquid suction port 16c. The reasons why the valve body 91a has the conical shape are that if the valve body 91a is inserted into the liquid suction port 16c of circular shape with its pointed side first, the two members are autonomously centered, that the flowrate can be fine adjusted by the degree of insertion, and that the sealability can be improved when the valve body 91a is fully inserted. When the valve body 91a is fully inserted, the flat surface around the bottom of the valve body 91a can be brought into close contact with the flat surface around the liquid suction port 16c to hermetically seal the liquid suction port 16c.

As shown in FIG. 26, if the liquid level of the liquid 2 rises and the floating member 92 floats up, the valve body 91a therefore moves away from the liquid suction port 16c to let the liquid 2 of the liquid receptor 15a into the receptor-side suction passage 16b. As a result, the receptor-side aerosol forming member 16 can suck the liquid 2 near the liquid surface of the liquid receptor 15a. This is convenient if the liquid 2 of the liquid receptor 15a is heated by the heater device 60, because the warm liquid 2 collecting to the liquid surface can be preferentially sucked.

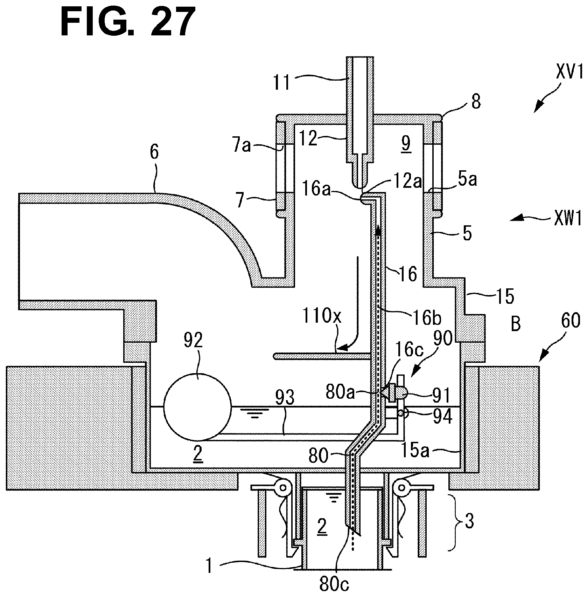

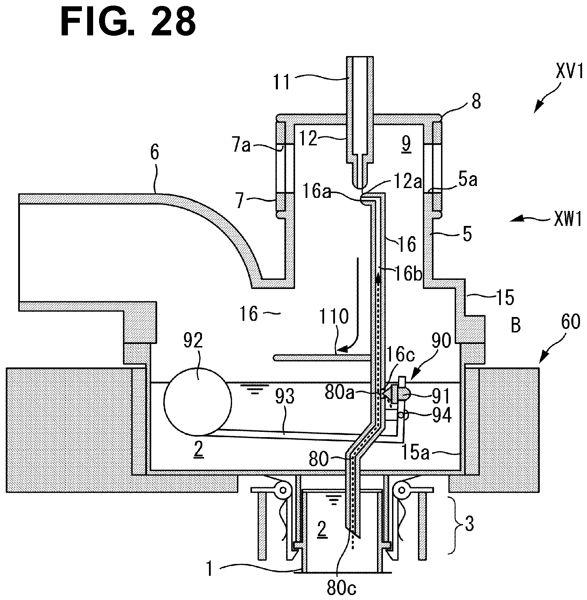

On the other hand, as shown in FIG. 27, if the liquid level of the liquid 2 falls and the floating member 92 lowers, the valve body 91a moves into the liquid suction port 16c and blocks the liquid suction port 16c. As a result, the negative suction pressure of the receptor-side aerosol forming member 16 is applied to the communication passage 80 side, and the liquid 2 in the water bottle 1 can be sucked. In fact, as shown in FIG. 28, the gap between the liquid suction port 16c and the valve body 91a autonomously comes to a balance at an optimum distance. The suction of the liquid 2 from the liquid suction port 16c and the suction of the liquid 2 in the water bottle 1 are simultaneously performed, to maintain a state where the consumption of the liquid 2 by humidification and the amount of the liquid 2 sucked from the water bottle 1 equal to each other.

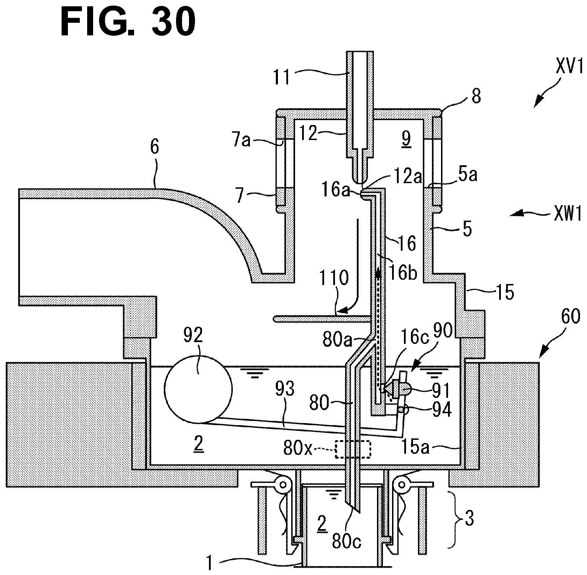

In the foregoing embodiment, the confluent port 80a of the communication passage 80 is described to be arranged under the liquid surface of the liquid 2 accumulated in the liquid receptor 15a. However, the present invention is not limited thereto, and the confluent port 80a may join the receptor-side suction passage 16b at any point. For example, like an application example shown in FIG. 30, the confluent port 80a may join above the liquid surface of the liquid 2. Such a configuration can reduce backflow of the liquid 2 of the liquid receptor 15a to the water bottle 1 via the liquid suction port 16c and the communication passage 80 when the device is stopped. It will be understood that as shown by the dotted lines in FIGs. 26 and 30, a check valve 80x may be provided on the communication passage 80 to suppress the backflow to the water bottle 1. It will be understood that the confluent port 80a may be arranged immediately before the ejection port 16a.

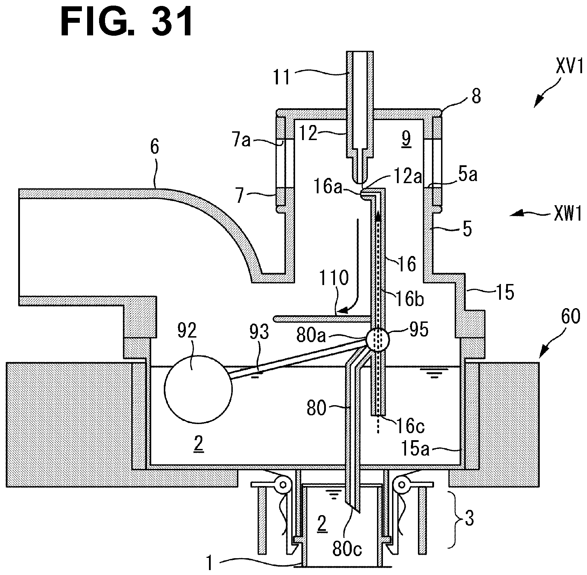

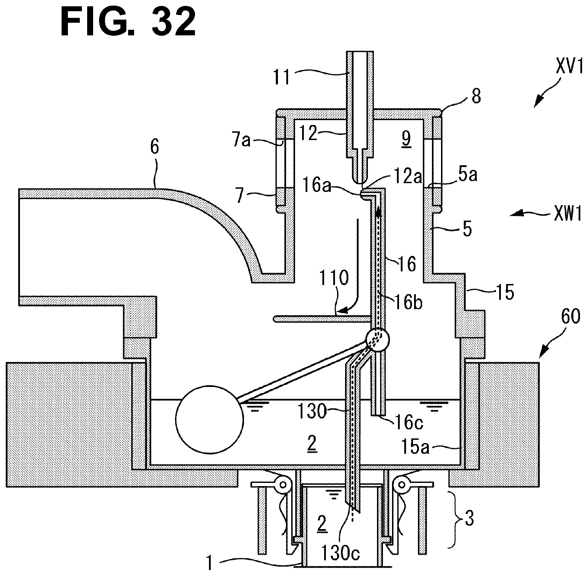

In the foregoing embodiment, the suction force control mechanism is described to adjust the suction force of the water bottle 1 by blocking the liquid suction port 16c. However, the present invention is not limited thereto. For example, like a suction force control mechanism shown in FIGs. 31 and 32, a three-way valve 95 may be arranged on the confluent port 80a of the communication passage 80. The three-way valve 95 may be rotated by the floating member 92 and the connection member 93 to switch between the state shown in FIG. 31, where the liquid level rises and the liquid suction port 16c and the ejection port 16a communicate to suck the liquid 2 of the liquid receptor 15a, and the state shown in FIG. 32, where the liquid level falls and the liquid suction port 80c of the communication passage 80 and the ejection port 16a communicate to suck the liquid 2 of the water bottle 1.

The present invention is not limited to the foregoing embodiments, and various modifications may be made without departing from the gist and technical idea thereof. The configuration of each embodiment or each modification may be applied to other embodiments and other modifications as far as possible.

More specifically, in the foregoing embodiments, the positions, sizes (dimensions), shapes, materials, directions, numbers, and the like of the components may be changed as appropriate.

In the foregoing third embodiment, the foregoing fourth embodiment, and the foregoing fifth embodiment, the valves 30, 40, and 50 are described to open and close the ejection port 13a of the container-side aerosol forming member 13. However, the present invention is not limited thereto, and the valves may be configured to open and close the outlet of the container-side suction member.

### Reference Signs List

- XA1, XD1, XF1, XH1, XJ1: nebulizer (humidifying device)
- XB1, XE1, XG1, XI1, XK1: nebulizer adaptor (adaptor)
- XC1: oxygen flowmeter
- 1: water bottle (container)
- 2: liquid such as sterile water
- 3: cap
- 4: gasket
- 5: erected projection portion
- 6: horizontal projection portion (sending unit)
- 7: adjustment dial
- 8: top plate
- 9: closed space
- 10: nut
- 11: terminal
- 12: nozzle member
- 12a: orifice
- 13: container-side aerosol forming member
- 14: diffuser
- 15: housing of nebulizer adaptor
- 15a: liquid receptor
- 16: receptor-side aerosol forming member
- 17: outlet member of oxygen flowmeter
- 23: container-side suction member
- 26: aerosol forming member
- 31: cutoff member
- 32: floating member
- 33: connection member
- 34: oscillation shaft

## Claims

1. A humidifying device comprising:
a suction mechanism configured to suck a liquid for humidification from a container containing the liquid by a negative pressure produced by a gas jetted from a gas jetting part of a nozzle member;
a liquid receptor configured to store the liquid sucked by the suction mechanism; and
a receptor-side aerosol forming member configured to suck the liquid from the liquid receptor and form an aerosol of the sucked liquid by the negative pressure by using a receptor-side suction passage.

2. The humidifying device according to claim 1, wherein
the suction mechanism includes a container-side suction member configured to suck the liquid from the container by the negative pressure produced by the gas jetted from the gas jetting part of the nozzle member.

3. The humidifying device according to claim 1, wherein:
the container-side suction member of the suction mechanism is a container-side aerosol forming member configured to suck the liquid from the container and form an aerosol of the sucked liquid by the negative pressure produced by the gas jetted from the gas jet part of the nozzle member; and
the liquid receptor stores at least the liquid discharged from the container-side aerosol forming member.

4. The humidifying device according to claim 1, comprising a suction force control mechanism configured to make capability of the suction mechanism to suck the liquid of the container variable by using a rise or fall of a liquid surface of the liquid stored in the liquid receptor.

5. The humidifying device according to claim 4, wherein:
the suction mechanism includes a discharge port configured to discharge the liquid sucked from the container; and
the suction force control mechanism includes
a cutoff member configured to move between a cutoff position in which to cut off the discharge port from a flow of the gas jetted from the gas jetting part and a retracted position in which to be retracted from the cutoff position,
a floating member configured to float up and down according to the rise or fall of the liquid surface of the liquid stored in the liquid receptor, and
a connection member configured to connect the floating member and the cutoff member and cause the cutoff member to move with floating of the floating member.

6. The humidifying device according to claim 5, wherein
the cutoff member moves between the cutoff position and the retracted position by oscillating about an oscillation shaft.

7. The humidifying device according to claim 4, wherein:
the suction mechanism includes a container-side suction passage configured to suck and guide the liquid from the container; and
the suction force control mechanism includes
an air opening port that is capable of opening a middle part of the container-side suction passage to an air side, and
an air control valve configured to open and close the air opening port according to the rise or fall of the liquid surface of the liquid stored in the liquid receptor.

8. The humidifying device according to claim 4, wherein:
the suction mechanism includes a container-side suction passage configured to suck and guide the liquid from the container; and
the suction force control mechanism includes a container-side suction passage control valve configured to control passing of the liquid of the container-side suction passage according to the rise or fall of the liquid surface of the liquid stored in the liquid receptor.

9. The humidifying device according to claim 1, wherein
the suction mechanism includes a communication passage configured to communicate the liquid of the container to the receptor-side suction passage of the receptor-side aerosol forming member, so that the suction mechanism sucks the liquid from the container by using a suction force of the receptor-side aerosol forming member.

10. The humidifying device according to claim 9, comprising a suction force control mechanism configured to make capability of the suction mechanism to suck the liquid of the container variable by using a rise or fall of a liquid surface of the liquid stored in the liquid receptor.

11. The humidifying device according to claim 10, wherein:
the suction force control mechanism includes a control member that is arranged on the receptor-side suction passage and configured to control a suction amount of the liquid of the liquid receptor by using the rise or fall of the liquid surface of the liquid stored in the liquid receptor; and
the suction force control mechanism accelerates suction of the liquid of the container through the communication passage by suppressing the suction amount of the liquid of the liquid receptor by the control member.

12. The humidifying device according to claim 11, wherein
the control member of the suction force control mechanism includes:
an opening and closing member configured to move between a closing position where a liquid suction port of the liquid of the liquid receptor in the receptor-side aerosol forming member is closed and an opening position where the liquid suction port is opened;
a floating member configured to float up and down according to the rise or fall of the liquid surface of the liquid stored in the liquid receptor; and
a connection member configured to connect the floating member and the opening and closing member and cause the opening and closing member to move with floating of the floating member.

13. The humidifying device according to claim 1, comprising a second liquid receptor configured to receive the liquid above a bottom surface of the liquid receptor, wherein
the receptor-side aerosol forming member sucks the liquid guided by the second liquid receptor and form an aerosol of the sucked liquid.

14. The humidifying device according to claim 13, wherein
the second liquid receptor is submerged in the liquid stored in the liquid receptor.

15. The humidifying device according to claim 13, wherein
the second liquid receptor also serves as a restriction member configured to restrict impingement of the gas jetted from the gas jetting part of the nozzle member on a liquid surface of the liquid receptor.

16. The humidifying device according to claim 1, comprising a heating mechanism configured to heat at least part of the liquid of the liquid receptor or the liquid in the receptor-side aerosol forming member.

17. The humidifying device according to claim 1, comprising an adaptor that is detachably attached to the container, wherein
the adaptor includes at least the receptor-side aerosol forming member, the liquid receptor, and the suction mechanism.

18. The humidifying device according to claim 1, the humidifying device humidifying and sending the gas containing oxygen to a patient, wherein
the humidifying device comprises a sending unit configured to send out a mixture of the gas and the aerosol.

19. The humidifying device according to claim 1, comprising the container.
